# EUROPÄISCHE PATENTSCHRIFT

(11) **EP 2 379 478 B1**
(45) Veröffentlichungstag und Bekanntmachung des Hinweises auf die Patenterteilung: **10.08.2016**
(21) Anmeldenummer: 09774888.3
(22) Anmeldetag: 10.12.2009
(51) Int. Cl.: C07C 29/19, C07C 31/135

(54) **KONTINUIERLICHES VERFAHREN ZUR HERSTELLUNG VON SUBSTITUIERTEN CYCLOHEXYLMETHANOLEN**
CONTINUOUS METHOD FOR PRODUCING SUBSTITUTED CYCLOHEXYLMETHANOLS
PROCÉDÉ CONTINU DE PRODUCTION DE CYCLOHEXYLMÉTHANOLS SUBSTITUÉS

(30) Priorität: 17.12.2008 EP 08171922
(43) Veröffentlichungstag der Anmeldung: 26.10.2011
(73) Patentinhaber: BASF SE, 67056 Ludwigshafen am Rhein (DE)
(72) Erfinder: MIRK, Daniela, 67346 Speyer (DE); KÖNIGSMANN, Lucia, 70197 Stuttgart (DE); HENKELMANN, Jochem, 68165 Mannheim (DE)
(86) Internationale Anmeldenummer: PCT/EP2009/066834
(87) Internationale Veröffentlichungsnummer: WO 2010/079035

(56) Entgegenhaltungen:
- EP-A2- 0 814 098
- EP-A2- 1 090 902
- WO-A2-2006/136541
- DE-A1- 2 427 609
- DE-A1- 3 537 228
- JP-A- 62 185 032
- KR-A- 20040 072 433

## Beschreibung

Die vorliegende Erfindung betrifft ein kontinuierliches Verfahren zur Herstellung von 1-Hydroxyalkyl-cyclohexanverbindungen, speziell von 4-Isopropylcyclohexyl-methanol, durch katalytische Hydrierung der entsprechenden Aldehyde oder Ketone in Gegenwart von Wasserstoff und eines geträgerten Ruthenium-Katalysators.

4-Alkyl- oder Alkenyl-substitierte Cyclohexylmethanole sowie deren Ether und Ester stellen wertvolle Aromachemikalien dar, die aufgrund ihres Maiglöckchen-artigen Duftes breite Anwendung in der Aromatisierung von Gebrauchs- oder Verbrauchsgütern aller Art finden. Eine in dieser Hinsicht besonders gefragte Verbindung ist das 4-Isopropylcyclohexylmethanol, das üblicherweise in Form eines Gemisches seines cis- und trans-Isomeren eingesetzt und unter dem Handelsnamen Mayol® (Firmenich SA, Genf) vertrieben wird.

Aufgrund der stetig zunehmenden Nachfrage der genannten Aromachemikalien, speziell von 4-Isopropylcyclohexylmethanol, kann diese durch die bestehenden Verfahren zur Herstellung der genannten Verbindungen immer schwerer gedeckt werden. Daher besteht Bedarf an einem leistungsfähigen und für die Herstellung im industriellen Maßstab geeigneten Herstellverfahren der genannten Verbindungen, insbesondere für die Herstellung von 4-Isopropylcyclohexylmethanol.

Die DE 24 27 609 offenbart alicyclische Cyclohexylmethanole und deren Ether oder Ester, die in 4-Position einen Isopropyl- oder Isopropenylrest aufweisen, sowie deren Verwendung als Riech- oder Geschmacksstoffe. Darüber hinaus offenbart die Schrift Verfahren zur Herstellung der genannten Verbindungen durch katalytische Hydrierung von entsprechend ungesättigten Ausgangsverbindungen. Beispielhaft wird die Herstellung von 4-Isopropylcyclohexylmethanol durch katalytische Hydrierung von Cuminaldehyd in 1,2-Dimethoxyethan als Lösungsmittel und in Gegenwart eines Ruthenium-Kohle-Katalysators mit einem Rutheniumgehalt von 5% beschrieben. Die Umsetzung wurde bei einem Druck von 100 Atmosphären und bei einer Temperatur von 130°C durchgeführt und lieferte nach fraktionierter Destillation des Rohproduktes 4-Isopropylcyclohexylmethanol in Form eines Gemisches von 70:30 Gewichtsteilen der cis- und trans-Isomeren.

Die EP 0 293 739 betrifft ein Verfahren zur Herstellung von 4-Isopropylcyclohexylmethanol bzw. dessen Alkylethern durch Kernhydrierung von 4-(1-Alkoxy-1-methyl-ethyl)-benzaldehyden bzw. deren Dialkylacetalen in Gegegenwart von Edelmetallen der Gruppe VIII des Periodensystems wie beispielsweise Nickel, Palladium, Platin, Rhodium und Ruthenium. Die genannten Metalle können auf ein Katalysatorträgermaterial wie beispielsweise Aluminiumoxid oder Aktivkohle in Mengen von 0,5 bis 10 Gew.-% aufgetragen sein oder auch in Form der reinen Metalle oder Metallverbindungen eingesetzt werden. Die Umsetzungen werden unter Wasserstoffdrücken von 50 bis 350 bar und bei Temperaturen von 100 bis 250°C durchgeführt.

Die EP 0 992 475 offenbart ein Verfahren zur Herstellung eines Alkohols durch katalytische Hydrierung des entsprechenden Aldehyds, ausgenommen 3-Hydroxypropionaldehyd, oder Ketons in wässriger oder organischer Lösung bei einer Temperatur von 20 bis 200°C und einem H₂-Druck von 0,5 bis 30 MPa unter Verwendung eines trägergebundenen Rutheniumkatalysators, das dadurch gekennzeichnet ist, dass man als Katalysator Ruthenium auf einem oxidischen Träger aus der Reihe TiO₂, SiO₂, ZrO₂, MgO, Mischoxide und Silikate, ausgenommen Zeolithe, mit einem Ru-Gehalt von 0,1 bis 20 Gew.-% verwendet. Durch die Verwendung von TiO₂ und SiO₂ als Trägermaterialien werden hohe Standzeiten des Katalysators erreicht, es wird allerdings keine Kernhydrierung aromatischer Substrate beschrieben.

Die EP 1 004 564 betrifft ein Verfahren zur Herstellung von Hydroxyethylcyclohexanen durch katalytische Hydrierung der entsprechenden Hydroxyethylbenzole mittels Ruthenium als Katalysator, das vor dem Einsatz mit einem Reduktionsmittel behandelt wurde. Das Verfahren wird in einem Alkan mit einem Siedepunkt von über 70°C als Lösungsmittel durchgeführt.

Die KR 20040072433 offenbart ein Verfahren zur Herstellung von 1-Cyclohexyl-1-ethanol durch kontinuierliche Hydrierung von 1-Methylbenzylalkohol und/oder Acetophenon an einem Kieselgel-geträgerten Ruthenium-Katalysator mit einem Ruthenium-Gehalt von 1 bis 5 Gew.-%, wobei der Kieselgelträger einen "acid-activity-index" von weniger als 10% aufweist.

Die EP 1 676 829 betrifft ein Verfahren zur kontinuierlichen katalytischen Hydrierung von hydrierbaren Verbindungen, speziell von aromatischen Carbonsäuren oder deren Derivaten, die kernhydriert werden. Die Umsetzung erfolgt an festen, im Festbett angeordneten Katalysatoren mit einem wasserstoffhaltigen Gas und ist dadurch gekennzeichnet, dass die Hydrierung in mindestens zwei hintereinandergeschalteten Hydriereinheiten durchgeführt wird und dass mindestens eine der beiden Hydriereinheiten in Schlaufenfahrweise betrieben wird und wobei für die Hydriereinheiten Katalysatorvolumina verwendet werden, die nach einem speziellen Bestimmungsverfahren erhältlich sind.

Die DE 35 37 228 A1 betrifft ein Verfahren zur Herstellung von Cyclohexylverbindungen durch katalytische Hydrierung von benzylischen Verbindungen, wobei man die benzylischen Verbindungen in Gegenwart eines Ru-Trägerkatalysators hydriert.

Die EP 1 090 902 A2 betrifft ein Verfahren zur Herstellung von Alkoholen wie Cyclohexandimethanol. Die Alkohole werden durch Hydrierung eines Benzolrings des zunächst hergestellten Benzylesters und anschließende Esterhydrolyse erhalten oder der Benzylester wird erst zum Benzylalkohol hydrolysiert und dann ein Benzolring des Benzylalkohols hydriert.

JP 62 185032 A betrifft ein Verfahren, in dem Alkylacetophenonderivate mit Wasserstoffgas in Gegenwart eines Ru-Katalysators in wässriger alkalischer Lösung reduziert werden.

Ausgehend von diesem Stand der Technik bestand die der vorliegenden Erfindung zu Grunde liegende Aufgabe darin, ein kontinuierliches Verfahren zur Herstellung von 4-Isopropylcyclohexylmethanol sowie eng verwandter Verbindungen bereitzustellen, das es ermöglicht, die gewünschte Zielverbindung
- ausgehend von möglichst wohlfeilen und gut verfügbaren Ausgangsstoffen,
- in möglichst hoher Ausbeute und hoher chemischer Reinheit,
- in möglichst diastereomerenangereicherter Form,
- mit einem möglichst hohen Gehalt des gewünschten cis-Isomeren,
- mit einem möglichst geringem und leicht abtrennbaren Nebenproduktspektrum,
- auf verfahrenstechnisch möglichst einfache Weise,
- unter Einsatz eines möglichst wohlfeilen Katalysators und
- unter möglichst weitgehendem Verzicht auf Lösemittel und andere Zusätze oder Reagenzien,
zu erhalten.

Die Aufgabe wurde erfindungsgemäß gelöst durch die Bereitstellung eines kontinuierlichen Verfahrens zur Herstellung eines 1-Hydroxyalkyl-cyclohexans der Formel (I) 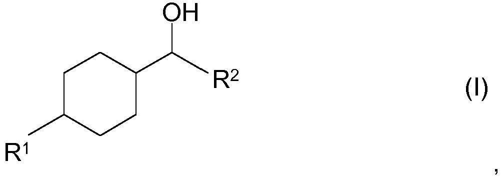 wobei die Reste
R¹ Isopropyl und
R² Wasserstoff oder einen geradkettigen oder verzweigten Alkylrest mit 1 bis 3 Kohlenstoffatomen
bedeuten,
durch katalytische Hydrierung einer aromatischen Carbonylverbindung der Formel (II) 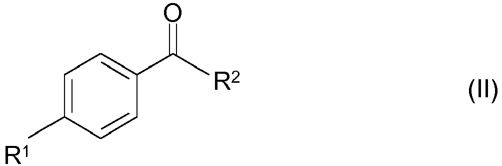 und/oder durch katalytische Hydrierung eines aromatischen Alkohols der Formel (III) 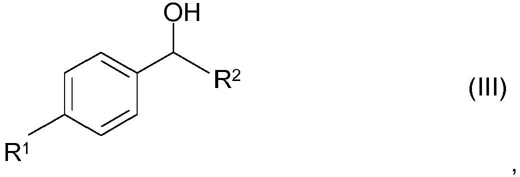 in denen die Reste R¹ und R² jeweils die gleiche Bedeutung wie in Formel (I) aufweisen,
in Gegenwart von Wasserstoff und in Gegenwart eines Katalysators, der als Aktivmetall Ruthenium aufgebracht auf einen Träger umfasst, wobei der Träger SiO₂ umfasst und wobei man den Katalysator in Form eines Festbettkatalysators in mehreren hintereinander geschalteten Hydrierreaktoren einsetzt, wobei der Festbettkatalysator bezogen auf das Gesamtgewicht des fertigen Katalysators einen Rutheniumgehalt von 0,1 bis 0,5 Gew.-% aufweist, wobei man die katalytische Hydrierung in einer Kaskade von n hintereinander geschalteten Hydrierreaktoren durchführt, wobei n eine ganze Zahl von 2 bis 5 bedeutet und wobei man
a1) das aus einem vorangehenden Hydrierreaktor kontinuierlich ausgetragene und nicht zurückgeführte Reaktionsgemisch und Wasserstoff kontinuierlich in den darauffolgenden Hydrierreaktor einträgt,
b1) das erhaltene, 1-Hydroxyalkyl-cyclohexan der Formel (I) enthaltende Reaktionsgemisch aus dem jeweiligen Hydrierreaktor kontinuierlich austrägt und gewünschtenfalls teilweise wieder in den jeweiligen Hydrierreaktor zurückführt und
c1) gewünschtenfalls das 1-Hydroxyalkyl-Cyclohexan der Formel (I) von dem in Schritt b1) aus dem n-ten Hydrierreaktor ausgetragenen und nicht wieder zurückgeführten Reaktionsgemisch abtrennt.

Als Ausgangsstoff zur Durchführung des erfindungsgemäßen Verfahrens können wahlweise die aromatischen Aldehyde oder Ketone der Formel (II) und/oder die aromatischen Alkohole der Formel (III) eingesetzt werden.

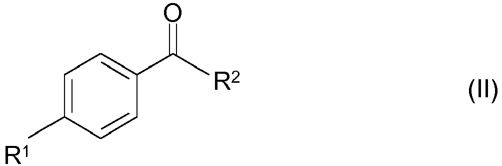

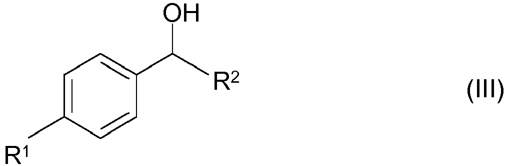

Die genannten aromatischen Ausgangsverbindungen tragen in para-Position des Aromaten einen iso-Propyl-Rest.

Der Rest R² kann in den erfindungsgemäß einsetzbaren Verbindungen der Formel (II) und (III) Wasserstoff oder einen geradkettigen oder verzweigten und Alkylrest mit 1 bis 3 Kohlenstoffatomen wie Methyl, Ethyl, n-Propyl oder iso-Propyl bedeuten. Bevorzugt bedeutet der Rest R² Wasserstoff.

Die genannten Verbindungen der Formeln (II) und (III) können wahlweise im Rahmen des erfindungsgemäßen Verfahrens eingesetzt werden, wobei bei jeweils gleicher Bedeutung der Reste R¹ und R² die gleiche Zielverbindung der Formel (I) erhalten wird, bei der die genannten Reste die gleichen Bedeutungen wie in der bzw. den Ausgangsverbindungen besitzen. Demgemäß können die Verbindungen der Formeln (II) und (III) bei gleicher Bedeutung der jeweiligen Reste R¹ und R² alleine, d.h. in reiner Form oder in Form von Gemischen untereinander eingesetzt werden.

Im Rahmen einer bevorzugten Ausführungsform führt man das erfindungsgemäße Verfahren lediglich unter Einsatz von aromatischen Carbonylverbindungen, d.h. Aldehyden oder Ketonen der Formel (II) durch. Wiederum bevorzugte Ausgangsverbindungen sind die Verbindungen der Formel (II), bei denen der Rest R² Wasserstoff bedeutet. Besonders bevorzugte Ausgangsverbindungen sind die para-substituierten Benzaldehyde der Formel (II), bei denen der Rest R² Wasserstoff und der Rest R¹ iso-Propyl bedeutet.

Eine erfindungsgemäß insbesondere bevorzugte Ausgangsverbindung ist demgemäß der para-Isopropylbenzaldehyd der Formel (IIa) 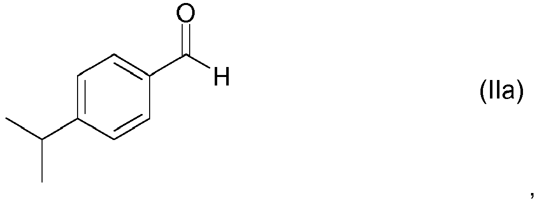 der auch als Cuminaldehyd bezeichnet wird.

Als Produkte des erfindungsgemäßen Verfahrens erhält man aus den gewählten Ausgangsverbindungen die 1-Hydroxyalkyl-cyclohexane der Formel (I) 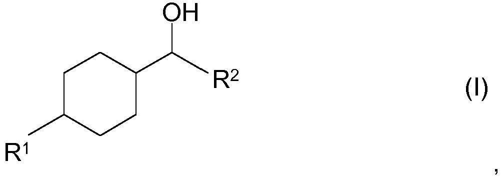 wobei die Reste R¹ und R² die gleichen Bedeutungen bzw. bevorzugten Bedeutungen aufweisen wie für die Formeln (II) und (III) beschrieben. Mögliche Verfahrensprodukte sind demnach die cycloaliphatischen Verbindungen der Formel (I), bei denen der Rest R¹ für iso-Propyl steht.

Der Rest R² kann in Formel (I) Wasserstoff oder einen geradkettigen oder verzweigten und Alkylrest mit 1 bis 3 Kohlenstoffatomen wie Methyl, Ethyl, n-Propyl oder iso-Propyl bedeuten. Bevorzugt bedeutet der Rest R² in Formel (I) Wasserstoff. Ein erfindungsgemäß besonders bevorzugtes Verfahrensprodukt ist daher das 4-Isopropylcyclohexyl-methanol der Formel (la)

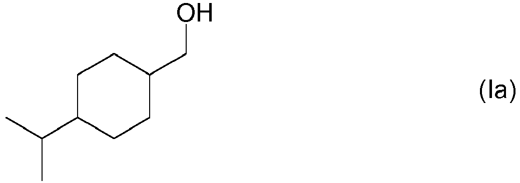

Die genannten cycloaliphatischen Verbindungen der Formeln (I) bzw. (la) können bezüglich der relativen Konfiguration der beiden Reste in 1- bzw. 4-Position des Cyclohexylringes in Form des trans-Isomers der Formel (Ib) 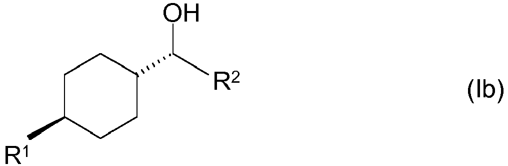 und in Form des cis-Isomeren der Formel (Ic) 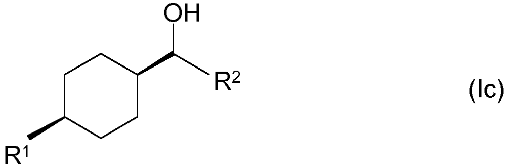 vorliegen. Die genannten Stereoisomeren werden erfindungsgemäß in der Regel in Form von Gemischen der jeweiligen cis- und trans-Isomere erhalten.

Im Rahmen des erfindungsgemäßen Verfahrens bleiben die Reste R¹ und R² der Ausgangsstoffe der Formeln (II) bzw. (III) erhalten, sofern sie nicht durch unerwünschte Nebenreaktionen beeinträchtigt werden. Es findet im Rahmen des erfindungsgemäßen Verfahrens lediglich die Hydrierung des aromatischen Kerns der gewählten Verbindung der Formel (II) und/oder (III) zum cycloaliphatischen Cyclohexylring statt sowie, bei Einsatz von Aldehyden oder Ketonen der Formel (II), eine Reduktion der Carbonylgruppe zur entsprechenden Alkoholgruppe.

Das erfindungsgemäße Verfahren wird in Gegenwart von Wasserstoff und in Gegenwart eines Katalysators, der als Aktivmetall Ruthenium aufgebracht auf einen Träger umfasst, wobei der Träger SiO₂ umfasst oder bevorzugt aus SiO₂ besteht, durchgeführt. Erfindungsgemäß einsetzbare Katalysatoren können Ruthenium als Aktivmetall alleine oder zusammen mit mindestens einem weiteren Metall der Nebengruppen IB, VIIB oder VIII des Periodensystems der Elemente (CAS-Version) umfassen. Derartige Katalysatoren sind bekannt und beispielsweise in den EP 0 814 098, WO 99/32427, WO 02/100536 sowie WO 2006/136541 auf die hiermit diesbezüglich ausdrücklich Bezug genommen wird, beschrieben.

Die erfindungsgemäß einzusetzenden Katalysatoren werden in Form eines Festbettkatalysators eingesetzt und weisen SiO₂ zumindest als Hauptbestandteile der Träger auf, wobei die genannten Trägermaterialien prinzipiell in allen dem Fachmann als geeigneten Formen oder Modifikationen eingesetzt werden kann. Die gewählten Trägermaterialien können dann in an sich bekannter Weise mit einer geeigneten Rutheniumverbindung und gegebenenfalls mit einer oder mehrer weiterer Verbindungen von Metallen der Nebengruppen IB, VIIB oder VIII des Periodensystems der Elemente behandelt werden. Derartige Herstellverfahren sind bekannt und umfassend beispielsweise in Catalyst preparation, Edited by J. Regalbuto, (2007), S. 177 oder Catalyst manufacture, A. B. Stiles, T. A. Koch, (1995), S. 377, sowie beispielsweise in der DE-A 101 282 05 und DE-A 101 282 42 sowie DE 196 244 85 beschrieben. Erfindungsgemäß führt man das erfindungsgemäße Verfahren in Gegenwart eines Katalysators, d.h. eines Festbettkatalysators durch, der lediglich SiO₂ als Träger umfasst.

Die erfindungsgemäß einzusetzenden Trägerkatalysatoren weisen einen RutheniumGehalt von 0,1 bis 0,5 Gew.-% auf (bezogen auf das Gesamtgewicht des fertigen, einsatzfähigen Katalysators).

Der Begriff Festbett ist im Rahmen der vorliegenden Erfindung breit aufzufassen wie beispielsweise in Römpp Online, Version 3.3, Georg Thieme Verlag, 2008 beschrieben. Demgemäß ist ein Festbett üblicherweise ein im Inneren eines Festbettreaktors fixierter, mit einem Katalysator versehener Träger großer Oberfläche. Der Reaktor wird üblicherweise durch zu reagierende Gase und/oder Flüssigkeiten (Fluide) durchströmt, die Reaktion findet am Katalysator (Kontakt) statt. Der Träger kann eine Schicht (Schüttung, Füllung) eines feinkörnigen Feststoffs (Trägermaterial: Körner, Kugeln, Pellets, etc.), ein Röhrenbündel, eine strukturierte Packung usw. sein. Weitere Erläuterungen finden sich z.B. auch unter G. Eigenberger, "Fixed-bed Reactors" in Ullmann's Encyclopedia of Industrial Chemistry 7th Edition (electronic encyclopedia).

Bei Einsatz eines Festbettkatalysators, der als Trägermaterial SiO₂ enthält, haben sich insbesondere die in der WO2006/136541 offenbarten Schalenkatalysatoren als vorteilhaft im Rahmen des erfindungsgemäßen Verfahrens erwiesen. Derartige Schalenkatalysatoren enthalten als Aktivmetall Ruthenium alleine oder zusammen mit mindestens einem weiteren Metall der Nebengruppen IB, VIIB oder VIII des Periodensystems der Elemente (CAS-Version), aufgebracht auf einen Träger enthaltend Siliziumdioxid als Trägermaterial.

Der genannte, erfindungsgemäß bevorzugt einsetzbare Schalenkatalysator ist dadurch gekennzeichnet, dass die Menge des Aktivmetalls < 1 Gew.-%, bevorzugt 0,1 bis 0,5 Gew.-%, besonders bevorzugt 0,25 bis 0,35 Gew.-%, bezogen auf das Gesamtgewicht des Katalysators, beträgt und mindestens 60 Gew.-%, besonders bevorzugt 80 Gew.- % des Aktivmetalls, bezogen auf die Gesamtmenge des Aktivmetalls, in der Schale des Katalysators bis zu einer Eindringtiefe von 200 µm vorliegen. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Weitere Informationen bezüglich den vorstehend genannten Meßverfahren und Techniken sind zum Beispiel "Spectroscopy in Catalysis" von J.W. Niemantsverdriet, VCH, 1995 offenbart.

Der erfindungsgemäß bevorzugt einsetzbare Schalenkatalysator zeichnet sich dadurch aus, dass die überwiegende Menge des Aktivmetalls in der Schale bis zu einer Eindringtiefe von 200 µm, also nahe der Oberfläche des Schalenkatalysators vorliegt. Dagegen liegt im Inneren (Kern) des Katalysators keine oder nur eine sehr geringe Menge des Aktivmetalls vor. Überraschenderweise wurde gefunden, dass der erfindungsgemäß bevorzugt einsetzbare Katalysator - trotz der geringen Menge an Aktivmetall - eine sehr hohe Aktivität bei der Hydrierung der erfindungsgemäß umzusetzenden Ausgangsverbindungen der Formeln (II) bzw. (III), bei sehr guten Selektivitäten, aufweist. Insbesondere nimmt die Aktivität des erfindungsgemäßen Katalysators über einen langen Hydrierzeitraum nicht ab.

Ganz besonders bevorzugt ist ein erfindungsgemäßer Schalenkatalysator, in dem kein Aktivmetall im Inneren des Katalysators nachgewiesen werden kann, d.h. Aktivmetall liegt nur in der äußersten Schale, zum Beispiel in einer Zone bis zu einer Eindringtiefe von 100 -200 µm, vor.

Der erfindungsgemäß bevorzugt einzusetzende Schalenkatalysator zeichnet sich in einer weiteren besonders bevorzugten Ausführungsform dadurch aus, dass im (FEG)-TEM (Field Emission Gun Transmission Electron Microscopy) mit EDXS nur in den äußersten 200 µm, bevorzugt 100 µm, ganz besonders bevorzugt 50 µm (Eindringtiefe) Aktivmetallteilchen nachgewiesen werden können. Teilchen kleiner 1 nm können nicht nachgewiesen werden.

Als Aktivmetall kann Ruthenium alleine oder zusammen mit mindestens einem weiteren Metall der Nebengruppen IB, VIIB oder VIII des Periodensystems der Elemente (CAS-Version) eingesetzt werden. Neben Ruthenium geeignete weitere Aktivmetalle sind z.B. Platin, Rhodium, Palladium, Iridium, Cobalt oder Nickel oder ein Gemisch aus zwei oder mehr davon. Unter den ebenfalls verwendbaren Metallen der Nebengruppen IB und/oder VIIB des Periodensystems der Elemente sind z.B. Kupfer und/oder Rhenium geeignet. Bevorzugt wird Ruthenium alleine als Aktivmetall oder zusammen mit Platin oder Iridium in dem erfindungsgemäßen Schalenkatalysator eingesetzt; ganz besonders bevorzugt wird Ruthenium alleine als Aktivmetall eingesetzt.

Der erfindungsgemäß einsetzbare Schalenkatalysator zeigt die vorstehend erwähnte sehr hohe Aktivität bei einer geringen Beladung mit Aktivmetall, die < 1 Gew.-%, bezogen auf das Gesamtgewicht des Katalysators, beträgt. Bevorzugt beträgt die Menge des Aktivmetalls in dem erfindungsgemäßen Schalenkatalysator 0,1 bis 0,5 Gew.-%, besonders bevorzugt 0,25 bis 0,35 Gew.-%. Es wurde gefunden, dass die Eindringtiefe des Aktivmetalls in das Trägermaterial von der Beladung des Katalysators mit Aktivmetall abhängig ist. Bereits bei einer Beladung des Katalysators mit 1 Gew.-% oder mehr, z.B. bei einer Beladung mit 1,5 Gew.-%, ist im Inneren des Katalysators, d.h. in einer Eindringtiefe von 300 bis 1000 µm eine wesentliche Menge Aktivmetall vorhanden, die die Aktivität des Hydrierkatalysators, insbesondere die Aktivität über einen langen Hydrierzeitraum, beeinträchtigt, insbesondere bei schnellen Reaktionen, wobei Wasserstoffmangel im Inneren des Katalysators (Kern) auftreten kann.

In dem erfindungsgemäß bevorzugt einzusetzenden Schalenkatalysator liegen mindestens 60 Gew.-% des Aktivmetalls, bezogen auf die Gesamtmenge des Aktivmetalls, in der Schale des Katalysators bis zu einer Eindringtiefe von 200 µm vor. Bevorzugt liegen in dem erfindungsgemäß einsetzbaren Schalenkatalysator mindestens 80 Gew.-% des Aktivmetalls, bezogen auf die Gesamtmenge des Aktivmetalls, in der Schale des Katalysators bis zu einer Eindringtiefe von 200 µm vor. Ganz besonders bevorzugt setzt man erfindungsgemäß einen Schalenkatalysator ein, in dem kein Aktivmetall im Inneren des Katalysators nachgewiesen werden kann, d.h. Aktivmetall liegt nur in der äußersten Schale, zum Beispiel in einer Zone bis zu einer Eindringtiefe von 100 - 200 µm, vor. In einer weiteren bevorzugten Ausführungsform liegen 60 Gew.-%, bevorzugt 80 Gew.-%, bezogen auf die Gesamtmenge des Aktivmetalls, in der Schale des Katalysators bis zu einer Eindringtiefe von 150 µm vor. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Zur Ermittlung der Eindringtiefe der Aktivmetallteilchen werden mehrere Katalysatorteilchen (z.B. 3, 4 oder 5) quer zur Strangachse (wenn der Katalysator in Form von Strängen vorliegt) angeschliffen. Mittels Linescans werden dann die Profile der Aktivmetall/Si Konzentrationsverhältnisse erfasst. Auf jeder Messlinie werden mehrere zum Beispiel 15-20 Messpunkte in gleichen Abständen gemessen; die Messfleckgröße beträgt circa 10 µm *10 µm. Nach Integration der Aktivmetallmenge über die Tiefe kann die Häufigkeit des Aktivmetalls in einer Zone bestimmt werden.

Ganz besonders bevorzugt beträgt die Menge des Aktivmetalls, bezogen auf das Konzentrationsverhältnis von Aktivmetall zu Si, an der Oberfläche des bevorzugt einzusetzenden Schalenkatalysators, 2 bis 25 %, bevorzugt 4 bis 10 %, besonders bevorzugt 4 bis 6 %, ermittelt mittels SEM EPMA - EDXS. Die Oberflächeanalyse erfolgt mittels Bereichsanalysen von Bereichen von 800 µm x 2000 µm und mit einer Informationstiefe von circa 2 µm. Die Elementzusammensetzung wird in Gew. % (normiert auf 100 %) bestimmt. Das mittlere Konzentrationsverhältnis (Aktivmetall/Si) wird über 10 Messbereiche gemittelt.

Unter der Oberfläche des Schalenkatalysators ist im Sinne der vorliegenden Anmeldung die äußere Schale des Katalysators bis zu einer Eindringtiefe von circa 2 µm zu verstehen. Diese Eindringtiefe entspricht der Informationstiefe bei der vorstehend erwähnten Oberflächenanalyse.

Ganz besonders bevorzugt setzt man einen Schalenkatalysator ein, worin die Menge des Aktivmetalls, bezogen auf das Gewichtsverhältnis von Aktivmetall zu Si (Gew./Gew. in %), an der Oberfläche des Schalenkatalysators 4 bis 6 % beträgt, in einer Eindringtiefe von 50 µm 1,5 bis 3 % und im Bereich von 50 bis 150 µm Eindringtiefe 0,5 bis 2 %, ermittelt mittels SEM EPMA (EDXS), beträgt. Die genannten Werte stellen gemittelte Werte dar.

Des Weiteren nimmt die Größe der Aktivmetallteilchen bevorzugt mit zunehmender Eindringtiefe ab, ermittelt mittels (FEG)-TEM-Analyse.

Das Aktivmetall liegt in dem erfindungsgemäßen Schalenkatalysator bevorzugt teilweise oder vollständig kristallin vor. In bevorzugten Fällen kann in der Schale des erfindungsgemäßen Schalenkatalysators mittels SAD (Selected Area Diffraction) oder XRD (X-Ray Diffraction) feinstkristallines Aktivmetall nachgewiesen werden.

Der erfindungsgemäß bevorzugt einsetzbare Schalenkatalysator kann zusätzlich Erdalkalimetallionen (M²⁺), also M = Be, Mg, Ca, Sr und/oder Ba, insbesondere Mg und/oder Ca, ganz besonders Mg enthalten. Der Gehalt an Erdalkalimetallion/en (M²⁺) im Katalysator beträgt bevorzugt 0,01 bis 1 Gew.-%, insbesondere 0,05 bis 0,5 Gew.- %, ganz besonders 0,1 bis 0,25 Gew.-%, jeweils bezogen auf das Gewicht des Siliziumdioxid-Trägermaterials.

Ein wesentlicher Bestandteil der erfindungsgemäß bevorzugt einsetzbaren Schalenkatalysatoren ist das Trägermaterial auf Basis von Siliziumdioxid, im Allgemeinen amorphem Siliziumdioxid. Unter dem Begriff "amorph" versteht man in diesem Zusammenhang, dass der Anteil kristalliner Siliziumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden.

Als Trägermaterialien kommen bei Einsatz der erfindungsgemäß bevorzugt einsetzbaren Schalenkatalysatoren grundsätzlich amorphe Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.-%, des Trägermaterials auch ein anderes oxidisches Material sein können, z.B. MgO, CaO, TiO₂, ZrO₂, Fe₂O₃ und/oderAlkalimetalloxid.

In einer bevorzugten Ausführungsform der Erfindung ist das Trägermaterial halogenfrei, insbesondere chlorfrei, d. h. der Gehalt an Halogen im Trägermaterial beträgt weniger als 500 Gew.-ppm, z.B. im Bereich von 0 bis 400 Gew.-ppm. Somit ist ein Schalenkatalysator bevorzugt, der weniger als 0,05 Gew.-% Halogenid (ionenchromatographisch bestimmt), bezogen auf das Gesamtgewicht des Katalysators, enthält.

Bevorzugt sind Trägermaterialien, die eine spezifische Oberfläche im Bereich von 30 bis 700 m²/g, vorzugsweise 30 bis 450 m²/g, (BET-Oberfläche nach DIN 66131) aufweisen.

Geeignete amorphe Trägermaterialien auf Basis von Siliziumdioxid sind dem Fachmann geläufig und kommerziell erhältlich (siehe z.B. O.W. Flörke, "Silica" in Ullmann's Encyclopedia of Industrial Chemistry 6th Edition on CD-ROM). Sie können sowohl natürlichen Ursprungs als auch künstlich hergestellt worden sein. Beispiele für geeignete amorphe Trägermaterialien auf Basis von Siliziumdioxid sind Kieselgele, Kieselgur, pyrogene Kieselsäuren und Fällungskieselsäuren. In einer bevorzugten Ausführungsform der Erfindung weisen die Katalysatoren Kieselgele als Trägermaterialien auf.

Je nach Ausgestaltung des erfindungsgemäß einzusetzenden Schalenkatalysators kann das Trägermaterial unterschiedliche Gestalt aufweisen. Bei Einsatz des erfindungsgemäßen Schalenkatalysators in Katalysatorfestbetten, wie im Rahmen der vorliegenden Erfindung, verwendet man üblicherweise Formkörper aus dem Trägermaterial, die z.B. durch Extrudieren, Strangpressen oder Tablettieren erhältlich sind und die z.B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 0,5 mm bis 25 mm. Häufig werden Katalysatorstränge mit Strangdurchmessern von 1,0 bis 5 mm und Stranglängen von 2 bis 25 mm eingesetzt. Mit kleineren Strängen können im Allgemeinen höhere Aktivitäten erzielt werden; jedoch zeigen diese oft keine ausreichende mechanische Stabilität im Hydrierverfahren. Daher werden ganz besonders bevorzugt Stränge mit Strangdurchmessern im Bereich von 1,5 bis 3 mm eingesetzt.

Die Herstellung der erfindungsgemäß bevorzugt einsetzbaren Schalenkatalysatoren erfolgt bevorzugt dadurch, dass man zunächst das Trägermaterial mit einer Lösung von Ruthenium(III)-Acetat alleine oder zusammen mit einer Lösung von mindestens einem weiteren Salz von Metallen der Nebengruppen IB, VIIB oder VIII des Periodensystems der Elemente (CAS-Version) ein- oder mehrfach tränkt, den erhaltenen Feststoff trocknet und anschließend reduziert, wobei die Lösung des mindestens einen weiteren Salzes von Metallen der Nebengruppen IB, VIIB oder VIII des Periodensystems der Elemente in einem oder mehreren gemeinsamen Tränkschritten gemeinsam mit der Lösung von Ruthenium(III)-Acetat oder in einem oder mehreren Tränkschritten getrennt von der Lösung von Ruthenium(III)-Acetat aufgebracht werden kann. Die einzelnen Verfahrensschritte sind nachstehend genauer beschrieben und umfassen die Schritte:
i) ein- oder mehrfache Tränkung des Trägermaterials enthaltend Siliziumdioxid mit einer Lösung von Ruthenium(III)-Acetat alleine oder zusammen mit einer Lösung von mindestens einem weiteren Salz von Metallen der Nebengruppen IB, VIIB oder VIII des Periodensystems der Elemente (CAS-Version);
ii) anschließendes Trocknen;
iii) anschließende Reduktion;
wobei die Lösung des mindestens einen weiteren Salzes von Metallen der Nebengruppen IB, VIIB oder VIII des Periodensystems der Elemente in einem oder mehreren Tränkschritten gemeinsam mit der Lösung von Ruthenium(III)-Acetat oder in einem oder mehreren Tränkschritten getrennt von der Lösung von Ruthenium(III)-Acetat aufgebracht werden kann.

### Schritt i)

In Schritt i) erfolgt eine ein- oder mehrfache Tränkung des Trägermaterials enthaltend Siliziumdioxid mit einer Lösung von Ruthenium(III)-Acetat alleine oder zusammen mit mindestens einem weiteren gelösten Salz von Metallen der Nebengruppen IB, VIIB oder VIII des Periodensystems der Elemente (CAS-Version). Da die Menge an Aktivmetall in dem erfindungsgemäßen Schalenkatalysator sehr gering ist, erfolgt in einer bevorzugten Ausführungsform eine einfache Tränkung. Ruthenium(III)-Acetat bzw. die Salze von Metallen der Nebengruppen IB, VIIB oder VIII des Periodensystems der Elemente stellen dabei Aktivmetallprekursor dar. Es wurde überraschenderweise gefunden, dass bei Verwendung von Ruthenium(III)-Acetat als Prekursor Schalenkatalysatoren erhalten werden können, die sich unter anderem dadurch auszeichnen, dass sich der wesentliche Teil des Aktivmetalls, bevorzugt Ruthenium alleine, in dem Schalenkatalysator bis zu einer Eindringtiefe von 200 µm befindet. Das Innere des Schalenkatalysators weist kein oder nur wenig Aktivmetall auf. Wird demgegenüber Ruthenium(III) -nitrosylnitrat als Prekursor eingesetzt, wie in den Beispielen in DE-A 101 28 205 und DE-A 101 28 242 offenbart, wird ein Rutheniumkatalysator erhalten, der Ruthenium homogen verteilt über den Katalysator bis zu schwach abgereichert im Inneren des Katalysators enthält.

Geeignete Lösungsmittel zur Bereitstellung der Lösung von Ruthenium(III)-Acetat bzw. der Lösung von mindestens einem weiteren Salz von Metallen der Nebengruppen IB, VIIB oder VIII des Periodensystems der Elemente sind Wasser oder auch Mischungen von Wasser bzw. Lösemittel mit bis zu 50 Vol.-% eines oder mehrerer mit Wasser bzw. Lösemittel mischbarer organischer Lösungsmittel, z.B. Mischungen mit C₁-C₄-Alkanolen wie Methanol, Ethanol, n-Propanol oder Isopropanol. Wässrige Essigsäure- oder Eisessig können ebenfalls verwendet werden. Alle Mischungen sollten so gewählt werden, dass eine Lösung oder Phase vorliegt. Bevorzugte Lösungsmittel sind Essigsäure, Wasser oder Mischungen hiervon. Besonders bevorzugt wird eine Mischung von Wasser und Essigsäure als Lösungsmittel eingesetzt, da Ruthenium(III)-Acetat üblicherweise gelöst in Essigsäure oder Eisessig vorliegt. Ruthenium(III)-Acetat kann jedoch auch als Feststoff nach Lösen verwendet werden. Der erfindungsgemäße Katalysator auch kann ohne Verwendung von Wasser hergestellt werden.

Die Lösung des mindestens einen weiteren Salzes von Metallen der Nebengruppen IB, VIIB oder VIII des Periodensystems der Elemente kann in einem oder mehreren Tränkschritten gemeinsam mit der Lösung von Ruthenium(III)-Acetat oder in einem oder mehreren Tränkschritten getrennt von der Lösung von Ruthenium(III)-Acetat aufgebracht werden. Das bedeutet, dass das Tränken mit einer Lösung erfolgen kann, die Ruthenium(III)-Acetat sowie mindestens ein weiteres Salz von Metallen der Nebengruppen IB, VIIB oder VIII des Periodensystems der Elemente aufweist. Das Tränken mit dieser Lösung kann ein- oder mehrfach erfolgen. Es ist jedoch ebenfalls möglich, dass zunächst mit einer Ruthenium(III)-Acetat- Lösung getränkt wird und anschließend in einem getrennten Tränkschritt mit einer Lösung, die mindestens ein weiteres Salz von Metallen der Nebengruppen IB, VIIB oder VIII des Periodensystems der Elemente aufweist, getränkt wird. Die Reihenfolge der Tränkschritte kann auch umgekehrt sein. Es ist ebenfalls möglich, dass einer der beiden Tränkschritte oder beide Tränkschritte einmal oder mehrfach in beliebiger Reihenfolge wiederholt werden. Nach jedem Tränkschritt wird üblicherweise getrocknet.

Geeignete Salze von weiteren Metallen der Nebengruppen IB, VIIB oder VIII des Periodensystems der Elemente, die in dem Tränkschritt eingesetzt werden können, sind z.B. Nitrate, Acetonate und Acetate, wobei Acetate bevorzugt sind.

Besonders bevorzugt erfolgt eine Tränkung mit einer Lösung von Ruthenium(III)-Acetat allein in einem Tränkschritt.

Das Tränken des Trägermaterials kann in unterschiedlicher Weise erfolgen und richtet sich in bekannter Weise nach der Gestalt des Trägermaterials. Beispielsweise kann man das Trägermaterial mit der Prekursor-Lösung besprühen oder spülen oder das Trägermaterial in der Prekursor-Lösung suspendieren. Beispielsweise kann man das Trägermaterial in einer wässrigen Lösung des Aktivmetallprekursors suspendieren und nach einer gewissen Zeit vom wässrigen Überstand abfiltrieren. Über die aufgenommene Flüssigkeitsmenge und die Aktivmetall-Konzentration der Lösung kann dann der Aktivmetallgehalt des Katalysators in einfacher Weise gesteuert werden. Das Tränken des Trägermaterials kann beispielsweise auch dadurch erfolgen, dass man den Träger mit einer definierten Menge der Lösung des Aktivmetallprekursors behandelt, die der maximalen Flüssigkeitsmenge entspricht, die das Trägermaterial aufnehmen kann. Zu diesem Zweck kann man beispielsweise das Trägermaterial mit der erforderlichen Flüssigkeitsmenge besprühen. Geeignete Apparaturen hierfür sind die zum Vermengen von Flüssigkeiten mit Feststoffen üblicherweise verwendeten Apparate (siehe Vauck/Müller, Grundoperationen chemischer Verfahrenstechnik, 10. Auflage, Deutscher Verlag für Grundstoffindustrie, 1994, S. 405 ff.), beispielsweise Taumeltrockner, Tränktrommeln, Trommelmischer, Schaufelmischer und dergleichen. Monolithische Träger werden üblicherweise mit den wässrigen Lösungen des Aktivmetallprekursors gespült.

Die zum Tränken eingesetzten Lösungen sind vorzugsweise halogenarm, insbesondere chlorarm, d.h. sie enthalten kein oder weniger als 500 Gew.-ppm, insbesondere weniger als 100 Gew.-ppm Halogen, z.B. 0 bis < 80 Gew.-ppm Halogen, bezogen auf das Gesamtgewicht der Lösung.

Die Konzentration des Aktivmetallprekursors in den Lösungen richtet sich naturgemäß nach der aufzubringenden Menge an Aktivmetallprekursor und der Aufnahmekapazität des Trägermaterials für die Lösung und beträgt < 20 Gew.-%, bevorzugt 0,01 bis 6 Gew.-%, besonders bevorzugt 0,1 bis 1,1 Gew.-%, bezogen auf die Gesamtmasse der eingesetzten Lösung.

### Schritt ii)

Das Trocknen kann nach den üblichen Verfahren der Feststofftrocknung unter Einhaltung der unten genannten Temperaturobergrenzen erfolgen. Die Einhaltung der Obergrenze der Trocknungstemperaturen ist für die Qualität, d.h. die Aktivität des Katalysators wichtig. Ein Überschreiten der unten angegebenen Trocknungstemperaturen führt zu einem deutlichen Verlust an Aktivität. Ein Kalzinieren des Trägers bei höheren Temperaturen, z.B. oberhalb 300°C oder gar 400°C, wie es im Stand der Technik vorgeschlagen wird, ist nicht nur überflüssig sondern wirkt sich auch nachteilig auf die Aktivität des Katalysators aus. Zur Erreichung hinreichender Trocknungsgeschwindigkeiten erfolgt die Trocknung bevorzugt bei erhöhter Temperatur, bevorzugt bei ≤ 180°C, besonders bei ≤ 160°C, und bei wenigstens 40°C, insbesondere wenigstens 70°C, speziell wenigstens 100°C, ganz besonders im Bereich von 110°C bis 150°C.

Die Trocknung des mit dem Aktivmetallprekursor getränkten Feststoffs erfolgt üblicherweise unter Normaldruck wobei zur Förderung der Trocknung auch ein verminderter Druck angewendet werden kann. Häufig wird man zur Förderung der Trocknung einen Gasstrom über bzw. durch das zu trocknende Gut leiten, z.B. Luft oder Stickstoff.

Die Trocknungsdauer hängt naturgemäß von dem gewünschten Grad der Trocknung und der Trocknungstemperatur ab und liegt bevorzugt im Bereich von 1 h bis 30 h, vorzugsweise im Bereich von 2 bis 10 h.

Vorzugsweise führt man die Trocknung des behandelten Trägermaterials soweit, dass der Gehalt an Wasser bzw. an flüchtigen Lösungsmittelbestandteilen vor der anschließenden Reduktion weniger als 5 Gew.-%, insbesondere nicht mehr als 2 Gew.-%, bezogen auf das Gesamtgewicht des Feststoffs ausmacht. Die angegebenen Gewichtsanteile beziehen sich hierbei auf den Gewichtsverlust des Feststoffs, bestimmt bei einer Temperatur von 160°C, einem Druck von 1 bar und einer Dauer von 10 Min. Auf diese Weise kann die Aktivität der erfindungsgemäß verwendeten Katalysatoren weiter gesteigert werden.

### Schritt iii)

Die Überführung des nach dem Trocknen erhaltenen Feststoffs in seine katalytisch aktive Form erfolgt durch Reduzieren des Feststoffs bei Temperaturen im Bereich von im Allgemeinen 150°C bis 450°C, bevorzugt 250°C bis 350°C in an sich bekannter Weise.

Zu diesem Zweck bringt man das Trägermaterial bei den oben angegebenen Temperaturen mit Wasserstoff oder einer Mischung aus Wasserstoff und einem Inertgas in Kontakt. Der Wasserstoffabsolutdruck ist für das Ergebnis der Reduktion von untergeordneter Bedeutung und kann z.B. im Bereich von 0,2 bar bis 1,5 bar variiert werden. Häufig erfolgt die Hydrierung des Katalysatormaterials bei Wasserstoffnormaldruck im Wasserstoffstrom. Vorzugsweise erfolgt die Reduktion unter Bewegen des Feststoffs, beispielsweise durch Reduzieren des Feststoffs in einem Drehrohrofen oder einem Drehkugelofen. Auf diese Weise kann die Aktivität der erfindungsgemäßen Katalysatoren weiter gesteigert werden. Der eingesetzte Wasserstoff ist vorzugsweise frei von Katalysatorgiften, wie CO- und S- enthaltenden Verbindungen, z.B. H₂S, COS und anderen.

Die Reduktion kann auch mittels organischer Reduktionsreagenzien wie Hydrazin, Formaldehyd, Formiaten oder Acetaten erfolgen.

Im Anschluss an die Reduktion kann der Katalysator zur Verbesserung der Handhabbarkeit in bekannter Weise passiviert werden, z.B. indem man den Katalysator kurzfristig mit einem Sauerstoff-haltigen Gas, z.B. Luft, vorzugsweise jedoch mit einer 1 bis 10 Vol.-% Sauerstoff enthaltenden Inertgasmischung, behandelt. Auch CO₂ oder CO₂/O₂-Mischungen können hier angewendet werden.

Der aktive Katalysator kann auch unter einem inerten organischen Lösungsmittel, z.B. Ethylenglykol, aufbewahrt werden.

Zur Herstellung des erfindungsgemäß bevorzugt einzusetzenden Schalenkatalysators kann in einer weiteren Durchführungsform der Aktivmetall-Katalysator-Vorläufer, z.B. wie oben hergestellt oder wie in WO-A2-02/100538 (BASF AG) beschrieben hergestellt, mit einer Lösung eines oder mehrerer Erdalkalimetall(II)salze imprägniert werden.

Bevorzugte Erdalkalimetall(II)salze sind entsprechende Nitrate, wie insbesondere Magnesiumnitrat und Calciumnitrat.

Bevorzugtes Lösungsmittel für die Erdalkalimetall(II)salze in diesem Imprägnierungsschritt ist Wasser. Die Konzentration des Erdalkalimetall(II)salzes im Lösungsmittel beträgt z.B. 0,01 bis 1 mol/Liter.

Z. B. wird der in einem Rohr eingebaute Aktivmetall/SiO₂-Katalysato mit einem Strom einer wässrigen Lösung des Erdalkalimetallsalzes kontaktiert. Der zu imprägnierende Katalysator kann auch mit einer überstehenden Lösung des Erdalkalimetallsalzes behandelt werden.

Bevorzugt findet so eine Sättigung des Aktivmetall/SiO₂-Katalysators insbesondere seiner Oberfläche, mit dem oder den Erdalkalimetallion/en statt.

Überschüssiges Erdalkalimetallsalz und nicht immobilisierte Erdalkalimetallionen wird/werden vom Katalysator gespült (H₂O-Spülung, Katalysatorwaschung).

Für vereinfachte Handhabung, z.B. Einbau in einem Reaktorrohr, kann der erfindungsgemäß bevorzugt einzusetzende Katalysator nach der Imprägnierung getrocknet werden. Die Trocknung kann dafür z.B. in einem Ofen bei < 200°C, z.B. bei 50 bis 190°C, besonders bevorzugt bei < 140°C, z. B. bei 60 bis 130°C, durchgeführt werden.

Dieses Imprägnierungsverfahren kann ex situ oder in situ durchgeführt werden: Ex situ heißt vor Einbau des Katalysators in den Reaktor, in situ bedeutet im Reaktor (nach dem Katalysatoreinbau).

In einer Verfahrensvariante kann eine Imprägnierung des Katalysators mit Erdalkalimetallionen auch in situ dadurch erfolgen, dass der Lösung des zu hydrierenden aromatischen Substrats (Edukts, hier den Verbindungen der Formeln (II) und/oder (III)) Erdalkalimetallionen, z.B. in Form von gelösten Erdalkalimetallsalzen, zugegeben werden. Dazu wird z.B. die entsprechende Menge Salz zunächst in Wasser gelöst und dann dem in einem organischen Lösungsmittel gelöstem Substrat zugegeben.

Herstellungsbedingt liegt das Aktivmetall in den erfindungsgemäß bevorzugt einsetzbaren Schalenkatalysatoren als metallisches Aktivmetall vor.

Durch die Verwendung halogenfreier, insbesondere chlorfreier, Aktivmetallprekursor und Lösungsmittel bei der Herstellung des erfindungsgemäß einsetzbaren Schalenkatalysators liegt der Halogenidgehalt, insbesondere Chloridgehalt, der erfindungsgemäßen Schalenkatalysatoren zudem unterhalb 0,05 Gew.-% (0 bis < 500 Gew.-ppm, z.B. im Bereich von 0 - 400 Gew.-ppm), bezogen auf das Gesamtgewicht des Katalysators.

Der Chloridgehalt wird z.B. mit der unten beschriebenen Methode ionenchromatographisch bestimmt.

In diesem Dokument sind alle ppm-Angaben als Gewichtsanteile zu verstehen (Gew.-ppm), soweit nichts anderes angegeben ist.

In einer ausgewählten Variante ist bevorzugt, dass das mittels ²⁹Si-Festkörper-NMR bestimmte prozentuale Verhältnis der Q₂ - und Q₃ - Strukturen Q₂/Q₃ kleiner als 25, bevorzugt kleiner als 20, besonders bevorzugt kleiner als 15 ist, z.B. im Bereich von 0 bis 14 oder 0,1 bis 13 liegt. Dies bedeutet auch, dass der Kondensationsgrad des Silikas in dem verwendeten Träger besonders hoch ist.

Die Identifikation der Qₙ - Strukturen (n = 2, 3, 4) und die Bestimmung des prozentualen Verhältnisses erfolgt mittels ²⁹Si-Festkörper-NMR.

Qn = Si(OSi)ₙ(OH)₄₋ₙ mit n = 1, 2, 3 oder 4.

Man findet Qₙ für n = 4 bei -110,8 ppm, n = 3 bei -100,5 ppm und n = 2 bei -90,7 ppm (Standard: Tetramethylsilan) (Qo und Q₁ wurden nicht identifiziert). Die Analyse wird unter den Bedingungen des "magic angle spinning" bei Raumtemperatur (20°C) (MAS 5500 Hz) mit Kreispolarisation (CP 5 ms) und unter Verwendung von dipolarer Entkopplung der ¹H durchgeführt. Wegen der teilweisen Überlagerung der Signale werden die Intensitäten über eine Linienformanalyse ausgewertet. Die Linienformanalyse wurde mit einem Standard Softwarepaket der Fa. Galactic Industries durchgeführt, wobei eine "least square fit" iterativ berechnet wurde.

Vorzugsweise enthält das Trägermaterial der erfindungsgemäß bevorzugt einsetzbaren SiO₂-Schalenkatalysatoren nicht mehr als 1 Gew.-% und insbesondere nicht mehr als 0,5 Gew.-% und insbesondere < 500 Gew.-ppm an Aluminiumoxid, gerechnet als Al₂O₃.

Da die Kondensation des Silikas auch durch Aluminium und Eisen beeinflusst werden kann, ist die Konzentration an Al(III) und Fe(II und/oder III) in Summe bevorzugt kleiner als 300 ppm, besonders bevorzugt kleiner 200 ppm, und liegt z.B. im Bereich von 0 bis 180 ppm.

Der Anteil an Alkalimetalloxid resultiert bevorzugt aus der Herstellung des Trägermaterials und kann bis zu 2 Gew.-% betragen. Häufig beträgt er weniger als 1 Gew.-%. Geeignet sind auch Alkalimetalloxid-freie Träger (0 bis < 0,1 Gew.-%). Der Anteil an MgO, CaO, TiO₂ bzw. an ZrO₂ kann bis zu 10 Gew.-% des Trägermaterials ausmachen und beträgt vorzugsweise nicht mehr als 5 Gew.-%. Geeignet sind aber auch Trägermaterialien, die keine nachweisbaren Mengen dieser Metalloxide enthalten (0 bis < 0,1 Gew.-%).

Weil Al(III) und Fe(II und/oder III) in Silica eingebaut acide Zentren ergeben können, ist es bevorzugt, dass eine Ladungskompensierung bevorzugt mit Erdalkalimetallkationen (M²⁺, M = Be, Mg, Ca, Sr, Ba) im Träger vorliegt. Dies bedeutet, dass das Gewichtsverhältnis von M(II) zu (Al(III) + Fe(II und/oder III)) größer ist als 0,5, bevorzugt > 1, besonders bevorzugt größer als 3.

Die römischen Zahlen in Klammern hinter dem Elementsymbol bedeuten die Oxidationsstufe des Elements.

Das erfindungsgemäße Verfahren wird auch in Gegenwart von Wasserstoff durchgeführt. Als Reaktionsgase kommen neben Wasserstoff auch wasserstoffhaltige Gase in Betracht, die keine Katalysatorgifte wie Kohlenmonoxid oder schwefelhaltige Gase wie H₂S oder COS enthalten, z.B. Mischungen von Wasserstoff mit Inertgasen wie Stickstoff oder Reformer-Abgase, die üblicherweise noch flüchtige Kohlenwasserstoffe enthalten. Bevorzugt setzt man reinen Wasserstoff (Reinheit ≥ 99,9 Vol.-%, besonders ≥ 99,95 Vol.-%, insbesondere ≥ 99,99 Vol.-%) ein. Dabei wird Wasserstoff oder das gewählte wasserstoffhaltige Gas in der Regel in jeden der eingesetzten Hydrierreaktoren ebenfalls kontinuierlich eingetragen.

Das erfindungsgemäße Verfahren zur Herstellung der cycloaliphatischen Verbindungen der Formel (I), bevorzugt von 4-Isopropylcyclohexylmethanol der Formel (la) bzw. (Ib) und (Ic) wird in Gegenwart von Wasserstoff und in Gegenwart eines Katalysators durchgeführt, der als Aktivmetall Ruthenium aufgebracht auf einen Träger umfasst, wobei der Träger SiO₂ umfasst und wobei man den Katalysator in Form eines Festbettkatalysators in mehreren hintereinander geschalteten Hydrierreaktoren einsetzt.

Das erfindungsgemäße Hydrierverfahren führt man in einer Kaskade von mehreren, nämlich 2 bis 5 hintereinander geschalteten Hydrierreaktoren durch.

Die erfindungsgemäße Hydrierung wird kontinuierlich durchgeführt. Als geeignete Reaktoren, d.h. Hydrierreaktoren seien Rieselreaktoren genannt oder solche, die in gefluteter Fahrweise (Sumpffahrweise) nach der Festbettfahrweise betrieben werden können. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung nach der Hydrierung am Katalysatorfestbett sind aus dem Stand der Technik bekannt, z.B. aus Ullmanns Enzyklopädie der Technischen Chemie, 4. Auflage, Band 13, S. 135 ff., sowie aus P. N. Rylander, "Hydrogenation and Dehydrogenation" in Ullmann's Encyclopedia of Industrial Chemistry, 5th ed. on CD-ROM.

Die erfindungsgemäße Hydrierung wird in einer Kaskade von mehreren, nämlich 2 bis 5, besonders bevorzugt 2 bis 4, ganz besonders bevorzugt 2 oder 3 und insbesondere bevorzugt 2 hintereinander geschalteten Hydrierreaktoren durchgeführt. Unter dem Begriff "hintereinander geschaltet" ist dabei sowie im Rahmen der gesamten vorliegenden Offenbarung zu verstehen, dass mehrere Hydrierreaktoren so miteinander verbunden sind, dass der Austrag des ersten bzw. eines vorangehenden Reaktors im Sinne einer Kaskaden- bzw. Reihenschaltung mit dem Eintrag des zweiten bzw. des jeweils unmittelbar darauffolgenden Hydrierreaktors verbunden ist.

Die genannten Reaktoren können dabei, wie vorstehend beschrieben, unabhängig voneinander in Rieselfahrweise oder in gefluteter Fahrweise (Sumpffahrweise) betrieben werden. Es ist auch möglich die in einer Kaskade hintereinander geschalteten Hydrierreaktoren in unterschiedlicher Fahrweise zu betreiben. Beispielsweise können der erste oder die ersten, bevorzugt die ersten beiden Hydrierreaktoren in Rieselfahrweise und der letzte Reaktor in Sumpffahrweise betrieben werden.

Die einzelnen Reaktoren können jeweils unabhängig voneinander unter vollständiger oder teilweiser Rückführung des aus dem Reaktor ausgetragenen Reaktionsgemisches betrieben werden. Im Rahmen einer bevorzugten Ausführungsform betreibt man zumindest einen der eingesetzten Hydrierreaktoren, besonders bevorzugt mindestens einen der in Rieselfahrweise betriebenen Hydrierreaktoren unter teilweiser Rückführung des ausgetragenen Reaktionsgemisches.

Die einzelnen Reaktoren können alternativ auch jeweils unabhängig voneinander im sogenannten geraden Durchgang, d.h. ohne vollständige oder teilweise Rückführung des aus dem Reaktor ausgetragenen Reaktionsgemisches betrieben werden. Im Rahmen einer bevorzugten Ausführungsform betreibt man zumindest einen der eingesetzten Hydrierreaktoren, besonders bevorzugt mindestens einen der in Sumpffahrweise betriebenen Hydrierreaktoren im sogenannten geraden Durchgang.

Im Rahmen einer bevorzugten Ausführungsform führt man das erfindungsgemäße Verfahren so durch, dass man den Katalysator in Form eines Festbettkatalysators in einem Hydrierreaktor oder mehreren hintereinander geschalteten Hydrierreaktoren einsetzt, wobei man
a) die Ausgangsverbindungen der der Formel (II) und/oder (III) sowie Wasserstoff kontinuierlich in den (ersten) Hydrierreaktor einträgt,
b) das erhaltene, 1-Hydroxyalkyl-cyclohexan der Formel (I) umfassende Reaktionsgemisch kontinuierlich aus dem (ersten) Hydrierreaktor austrägt und gewünschtenfalls teilweise wieder in den (ersten) Hydrierreaktor zurückführt und
c) gewünschtenfalls das 1-Hydroxyalkyl-Cyclohexan der Formel (I) von dem in Schritt b) aus dem (ersten) Hydrierreaktor ausgetragenen und nicht wieder zurückgeführten Reaktionsgemisch abtrennt.

Das erfindungsgemäße Verfahren wird kontinuierlich durchgeführt, wobei man in einem ersten Schritt a) die Ausgangsverbindung bzw. Ausgangsverbindungen der Formel (II) und/oder (III), bevorzugt Cuminaldehyd der Formel (IIa) sowie Wasserstoff kontinuierlich in einen ersten Hydrierreaktor einträgt, d.h. diesem kontinuierlich zuführt. Der Hydrierreaktor enthält den wie vorstehend beschriebenen Festbettkatalysator und kann wie vorstehend beschrieben in Sumpf- oder in Rieselfahrweise, bevorzugt in Rieselfahrweise betrieben werden.

Auf diese Weise erfolgt die vollständige oder teilweise Umsetzung zum gewünschten Reaktionsprodukt der Formel (I). Dieses wird in Form eines Reaktionsgemisches, das gegebenenfalls noch nicht umgesetztes Edukt oder auch nicht vollständig hydrierte Zwischenprodukte umfasst, gemäß Verfahrensschritt b) kontinuierlich aus dem Hydrierreaktor bzw. aus dem ersten Hydrierreaktor ausgetragen. Gewünschtenfalls kann das aus dem Hydrierreaktor bzw. aus dem ersten Hydrierreaktor gemäß Schritt b) ausgetragene, 1-Hydroxyalkyl-cyclohexan der Formel (I), bevorzugt 4-Isopropylcyclohexyl-methanol der Formel (la) umfassende Reaktionsgemisch teilweise wieder in diesen zurückgeführt werden, d.h. der jeweilige Hydrierreaktor kann auch im "Umlauf" oder in "Schlaufenfahrweise" betrieben werden.

Es ist demgemäß möglich, einen Teil des ausgetragenen Reaktionsgemisches wieder in denselben Hydrierreaktor zurückzuführen und den übrigen Teil kontinuierlich in einen weiteren Hydrierreaktor einzutragen. Auf diese Weise ist es möglich, insbesondere bei unterschiedlichen Reaktionsbedingungen in den einzelnen Hydrierreaktoren, den Reaktionsverlauf in gewünschter Weise zu steuern bzw. zu beeinflussen.

Gemäß dem optionalen Verfahrenschritt c) trennt man gewünschtenfalls das 1-Hydroxyalkyl-Cyclohexan der Formel (I) von dem in Schritt b) aus dem bzw. au dem ersten Hydrierreaktor ausgetragenen und nicht wieder zurückgeführten Reaktionsgemisch ab. Auf diese Weise können die gewünschten Zielverbindungen in hoher Reinheit erhalten werden. Die Abtrennung der gewünschten Zielverbindung der Formel (I), bevorzugt 4-Isopropylcyclohexylmethanol der Formel (la) kann dabei beispielsweise durch geeignete Destillationsverfahren, wie sie dem Fachmann bekannt sind, durchgeführt werden. Bei vollständiger bzw. sehr weitgehender Hydrierung der eingesetzten Ausgangsstoffe sowie bei geringem Ausmaß an Bildung von Nebenprodukten und in Abwesenheit von Lösemitteln erhält man bereits in Schritt b) ein Reaktionsgemisch bzw. -produkt, das in zu einem hohen Anteil, oft zu 90 Gew.-% oder darüber, bevorzugt zu 95 Gew.-% oder darüber, besonders bevorzugt zu 95 bis 99,5 Gew.-% aus dem gewünschten 1-Hydroxyalkyl-Cyclohexan der Formel (I) besteht. In diesen Fällen kann gewünschtenfalls von der optionalen Abtrennung gemäß Schritt c) abgesehen werden.

Das erfindungsgemäße Hydrierverfahren nimmt man so vor, dass man die katalytische Hydrierung in einer Kaskade von n hintereinander geschalteten Hydrierreaktoren durchführt, wobei n eine ganze Zahl von 2 bis 5 bedeutet und wobei man
a1)das aus einem vorangehenden Hydrierreaktor kontinuierlich ausgetragene und nicht zurückgeführte Reaktionsgemisch und Wasserstoff kontinuierlich in den darauffolgenden Hydrierreaktor einträgt,
b1) das erhaltene, 1-Hydroxyalkyl-cyclohexan der Formel (I) enthaltende Reaktionsgemisch aus dem jeweiligen Hydrierreaktor kontinuierlich austrägt und gewünschtenfalls teilweise wieder in den jeweiligen Hydrierreaktor zurückführt und
c1) gewünschtenfalls das 1-Hydroxyalkyl-Cyclohexan der Formel (I) von dem in Schritt b1) aus dem n-ten Hydrierreaktor ausgetragenen und nicht wieder zurückgeführten Reaktionsgemisch abtrennt.

Es versteht sich, dass man dabei, wie vorstehend unter Schritt a) beschrieben, die Ausgangsverbindungen der Formel (II) und/oder (III) sowie Wasserstoff kontinuierlich in den ersten Hydrierreaktor einträgt und, wie vorstehend unter Schritt b) beschrieben, das erhaltene, 1-Hydroxyalkyl-cyclohexan der Formel (I) umfassende Reaktionsgemisch kontinuierlich aus dem ersten Hydrierreaktor austrägt und gewünschtenfalls teilweise wieder in den (ersten) Hydrierreaktor zurückführt.

Bevorzugt bedeutet dabei der Index n eine ganze Zahl von 2 bis 4, besonders bevorzugt 2 oder 3 und insbesondere bevorzugt 2, entsprechend 2 bis 4, besonders bevorzugt 2 oder 3 und insbesondere bevorzugt 2 hintereinander geschalteten Hydrierreaktoren. Unter dem Begriff "vorangehenden Hydrierreaktor" ist wahlweise der erste oder ein anderer Hydrierreaktor in der Kaskade von n Hydrierreaktoren, mit Ausnahme des n-ten, d.h. letzten Hydrierreaktors der Kaskade, zu verstehen. Unter dem Begriff "darauffolgender Hydrierreaktor" ist der unmittelbar auf einen vorangehenden Reaktor folgende Hydrierreaktor zu verstehen. Demgemäß wäre der zweite Hydrierreaktor der Reihe von Hydrierreaktoren als "darauffolgender" Hydrierreaktor des ersten Hydrierreaktors zu verstehen. Der dritte Hydrierreaktor wäre als "darauffolgender" Hydrierreaktor des zweiten (dem vorangehenden Hydrierreaktor) Hydrierreaktors zu verstehen. Demgemäß können alle n Hydrierreaktoren wahlweise unter teilweiser Rückführung des erhaltenen Reaktionsgemisches, d.h. im Umlauf bzw. in Schlaufenfahrweise betrieben werden. Dabei können die einzelnen Hydrierreaktoren der Reaktorkaskade wahlweise jeweils in Sumpf- oder Rieselfahrweise sowie wahlweise mit oder ohne Rückführung des erhaltenen Reaktionsgemischen betrieben werden.

Erfindungsgemäß trägt man gemäß Schritt a1) das aus einem vorangehenden Hydrierreaktor, speziell dem ersten Hydrierreaktor, kontinuierlich ausgetragene und nicht zurückgeführte Reaktionsgemisch und Wasserstoff kontinuierlich in den darauffolgenden Hydrierreaktor ein. Gemäß Schritt b1) trägt man das erhaltene, 1-Hydroxyalkyl-cyclohexan der Formel (I) enthaltende Reaktionsgemisch aus dem jeweiligen Hydrierreaktor kontinuierlich aus und führt es gewünschtenfalls teilweise wieder in den jeweiligen Hydrierreaktor zurück. Gemäß Schritt c1) trennt man gewünschtenfalls das 1-Hydroxyalkyl-Cyclohexan der Formel (I) von dem in Schritt b1) aus dem n-ten d.h. letzten Hydrierreaktor der Kaskade ausgetragenen und nicht wieder zurückgeführten Reaktionsgemisch ab.

Alle der in der erfindungsgemäß bevorzugt einzusetzenden Kaskade vorgesehenen Hydrierreaktoren werden demnach vorzugsweise so betrieben, dass man den gewählten Ausgangsstoff bzw. das in einem vorangehenden Hydrierreaktor erhaltenen Reaktionsgemisch kontinuierlich in den jeweiligen Hydrierreaktor einträgt und das gebildete Reaktionsgemisch kontinuierlich wieder aus diesem austrägt.

Es hat sich darüber hinaus als vorteilhaft erwiesen, dass man den ersten Hydrierreaktor der Reaktorkaskade, d.h. den Reaktor, in den die gewählten Ausgangsstoffe der Formeln (II) und/oder (III) eingetragen werden, in Rieselfahrweise betreibt.

Darüber hinaus hat es sich als vorteilhaft erwiesen, dass man den weiteren Hydrierreaktor oder mindestens einen der weiteren Hydrierreaktoren in Sumpffahrweise betreibt. Insbesondere bevorzugt betreibt man den letzten Hydrierreaktor der Kaskade der hintereinandergeschalteten Hydrierreaktoren in Sumpffahrweise.

Das aus diesem letzten Hydrierreaktor ausgetragenen Reaktionsgemisch besteht häufig, wie vorstehend beschrieben, zu einem hohen Anteil aus den gewünschten 1-Hydroxyalkyl-Cyclohexan der Formel (I) bzw. bei Einsatz von Cuminaldehyd der Formel (IIa) aus 4-Isopropylcyclohexylmethanol der Formel (la). Dadurch kann sich der optionale Schritt der Abtrennung des Verfahrensproduktes aus dem erhaltenen Reaktionsgemisch je nach Anforderungen an die Reinheit des gewünschten Produktes erübrigen.

Im Prinzip ist es möglich, beim erfindungsgemäßen Einsatz einer Kaskade von zwei oder mehr Hydrierreaktoren diese auch mit verschiedenen der wie vorstehend beschriebenen geträgerten Ru-Festbettkatalysatoren zu betreiben. Es hat sich jedoch als praktikabel und vorteilhaft erwiesen, wenn der weitere Hydrierreaktor oder die weiteren Hydrierreaktoren den gleichen Festbettkatalysator enthält bzw. enthalten wie der erste Hydrierreaktor. Bevorzugt enthalten alle Reaktoren der Reaktorkaskade den gleichen wie vorstehend beschriebenen Ru auf einem SiO₂-Träger umfassenden Katalysator.

Im Rahmen einer bevorzugten Ausführungsform führt man das erfindungsgemäße Verfahren in einer Kaskade von 2 Hydrierreaktoren durch, von denen der erste (Hauptreaktor) in Rieselfahrweise betrieben wird. Der zweite, dem Hauptreaktor nach geschaltete Hydrierreaktor (Nachreaktor) kann wahlweise in Riesel- oder in Sumpffahrweise betrieben werden. Bevorzugt betreibt man den Nachreaktor in Sumpffahrweise. Wiederum bevorzugt werden der Hauptreaktor im Umlauf, d.h. unter teilweiser Rückführung des ausgetragenen Reaktionsgemisches und der Nachreaktor im geraden Durchgang betrieben, d.h. ohne Rückführung des ausgetragenen Reaktionsgemisches.

In einer weiteren bevorzugten Ausführungsform führt man das erfindungsgemäße Verfahren in einer Kaskade von 3 Hydrierreaktoren durch, von denen der erste und zweite (Hauptreaktor) in Rieselfahrweise betrieben werden und der dritte in Sumpffahrweise. Wiederum bevorzugt werden die ersten beiden Reaktoren im Umlauf, d.h. unter teilweiser Rückführung des jeweils ausgetragenen Reaktionsgemisches und der Nachreaktor im geraden Durchgang, d.h. ohne Rückführung des ausgetragenen Reaktionsgemisches betrieben.

Die eigentliche Hydrierung erfolgt üblicherweise in Analogie zu den bekannten Hydrierverfahren zur Hydrierung von organischen Verbindungen, die hydrierbare Gruppen aufweisen, bevorzugt zur Hydrierung einer carbocyclischen aromatischen Gruppe zu der entsprechenden carbocyclischen aliphatischen Gruppe (einer sogenannten Kernhydrierung), wie sie im eingangs genannten Stand der Technik beschrieben werden. Hierzu wird die organische Verbindung als flüssige Phase oder Gasphase, bevorzugt als flüssige Phase, mit dem Festbettkatalysator in Gegenwart von Wasserstoff in Kontakt gebracht.

Die erfindungsgemäße Hydrierung kann sowohl bei Wasserstoffnormaldruck als auch bei erhöhtem Wasserstoffdruck, z.B. bei einem Wasserstoffabsolutdruck von wenigstens 1,1 bar, vorzugsweise wenigstens 2 bar durchgeführt werden. Im Allgemeinen wird der Wasserstoffabsolutdruck einen Wert von 325 bar und vorzugsweise 300 bar nicht überschreiten. Besonders bevorzugt liegt der Wasserstoffabsolutdruck im Bereich von 10 bis 300 bar, ganz besonders bevorzugt im Bereich von 50 bis 250 bar und ganz besonders bevorzugt im Bereich von 100 bis 250 bar, insbesondere bevorzugt im Bereich von 125 bis 250 bar und am meisten bevorzugt im Bereich von 125 bis 200 bar (jeweils absolut).

Das erfindungsgemäße Hydrierverfahren kann in Abwesenheit eines Lösungs- oder Verdünnungsmittels oder auch in Gegenwart eines Lösungs- oder Verdünnungsmittels durchgeführt werden, d.h. es ist nicht erforderlich, die Hydrierung in Lösung durchzuführen.

Als Lösungs- oder Verdünnungsmittel kann jedes geeignete Lösungsmittel- oder Verdünnungsmittel eingesetzt werden. Als Lösungs- oder Verdünnungsmittel kommen grundsätzlich solche in Betracht, die die zu hydrierende organische Verbindung möglichst vollständig zu lösen vermögen oder sich mit dieser vollständig mischen und die unter den Hydrierungsbedingungen inert sind, d.h. nicht hydriert werden.

Beispiele für geeignete Lösungsmittel sind cyclische und acyclische Ether, z.B. Tetrahydrofuran, Dioxan, Methyl-tert.-butylether, Dimethoxyethan, Dimethoxypropan, Dimethyldiethylenglykol, aliphatische Alkohole wie Methanol, Ethanol, n- oder Isopropanol, n-, 2-, iso- oder tert.-Butanol, Carbonsäureester wie Essigsäuremethylester, Essigsäureethylester, Essigsäurepropylester oder Essigsäurebutylester, sowie aliphatische Etheralkohole wie Methoxypropanol und cycloaliphatische Verbindungen wie Cyclohexan, Methylcyclohexan und Dimethylcyclohexan.

Die Menge des gewünschtenfalls eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 3 bis 70 gew.-%igen Lösung der zur Hydrierung vorgesehenen organischen Verbindung führen.

Bei Verwendung eines Lösungsmittels wird im Rahmen des erfindungsgemäßen Verfahrens besonders bevorzugt das bei der Hydrierung gebildete Produkt, also bevorzugt die Verbindungen der Formel (I) als Lösungsmittel eingesetzt, gegebenenfalls neben anderen Lösungs- oder Verdünnungsmitteln oder auch neben Zwischenprodukten oder Nebenprodukten der Hydrierung wie z.B. p-Cymol, Cumol, 1-Isopropyl-4-methyl-cyclohexan (cis/trans) und Isopropylcyclohexan. In jedem Fall kann ein Teil des im Verfahren gebildeten Produkts dem noch zu hydrierenden Aromaten beigemischt werden.

Im Rahmen des erfindungsgemäßen Verfahrens kann auf den Zusatz weiterer, wie vorstehend beschriebener Lösungsmittel verzichtet werden. Eine bevorzugte Ausführungsform des erfindungsgemäßen Hydrierverfahrens ist demgemäß dadurch gekennzeichnet, dass man die Hydrierung ohne Zusatz von Lösungsmitteln (neben den genannten Zwischen- bzw. Nebenprodukten) durchführt.

Die Reaktionstemperaturen betragen im erfindungsgemäßen Verfahren im Allgemeinen wenigstens 30°C und werden häufig einen Wert von 250°C nicht überschreiten. Bevorzugt führt man das erfindungsgemäße Hydrierverfahren bei Temperaturen im Bereich von 50 bis 200°C, besonders bevorzugt 70 bis 200°C, und ganz besonders bevorzugt im Bereich von 80 bis 180°C und insbesondere bevorzugt im Bereich von 80 bis 160°C, noch mehr bevorzugt bis 140°C durch.

Dabei versteht es sich, dass im Falle des erfindungsgemäßen Einsatzes mehrerer hintereinander geschalteter Hydrierreaktoren diese bei unterschiedlichen Drucken und unterschiedlichen Temperaturen betrieben werden können.

Aufgrund der hohen Katalysatoraktivität benötigt man vergleichsweise geringe Mengen an Katalysator bezogen auf das eingesetzte Edukt. Bei der erfindungsgemäßen kontinuierlicher Ausgestaltung des Hydrierverfahrens wird man üblicherweise das zu hydrierende Edukt der Formel (II) oder (III) bzw. das aus einem vorangehenden Hydrierreaktor ausgetragene Reaktionsgemisch in einer Menge von 0,05 bis 3 kg/(I(Katalysator)•h), bevorzugt von 0,1 bis 2 kg/(I(Katalysator)•h), insbesondere bevorzugt von 0,1 bis 1,0 kg/(I(Katalysator)•h) und ganz besonders bevorzugt von 0,1 bis 0,6 kg/(I(Katalysator)•h) über den Katalysator führen.

Selbstverständlich können die in diesem Verfahren eingesetzten Katalysatoren bei nachlassender Aktivität nach den für Edelmetallkatalysatoren wie Rutheniumkatalysatoren üblichen, dem Fachmann bekannten Methoden regeneriert werden. Hier sind z.B. die Behandlung des Katalysators mit Sauerstoff wie in der BE 882 279 beschrieben, die Behandlung mit verdünnten, halogenfreien Mineralsäuren, wie in der US 4,072,628 beschrieben, oder die Behandlung mit Wasserstoffperoxid, z. B. in Form wässriger Lösungen mit einem Gehalt von 0,1 bis 35 Gew.-%, oder die Behandlung mit anderen oxidierenden Substanzen, vorzugsweise in Form halogenfreier Lösungen zu nennen. Üblicherweise wird man den Katalysator nach der Reaktivierung und vor dem erneuten Einsatz mit einem Lösungsmittel, z. B. Wasser, spülen.

Betreibt man einen oder mehrere der erfindungsgemäß eingesetzten Hydrierreaktoren unter teilweiser Rückführung des jeweils ausgetragenen Reaktionsgemisches, d.h. im Umlauf bzw. in Schlaufenfahrweise beträgt das Mengenverhältnis von eingetragener Stoffmenge zu rückgeführter Stoffmenge in den jeweiligen Hydrierreaktoren (Umlauf/Zulauf) etwa 1: 1 bis etwa 20:1, bevorzugt etwa 2:1 bis etwa 15:1 und besonders bevorzugt etwa 3:1 bis etwa 8:1.

Bei Einsatz der erfindungsgemäß als Ausgangsstoffe bevorzugten Aldehyde, d.h. bei Einsatz der Verbindungen der Formel (II), bei denen der Rest R² Wasserstoff bedeutet, besonders bevorzugt bei Einsatz von Cuminaldehyd der Formel (IIa) hat es sich als vorteilhaft erwiesen, wenn diese eine möglichst geringe Säurezahl im Bereich von 0 bis 5 mg KOH/g, bevorzugt bis zu 4 und besonders bevorzugt im Bereich von 0 bis 3 mg KOH/g aufweisen. Bei erfindungsgemäß einzusetzenden Aldehyden der Formel (II), die eine höhere Säurezahl aufweisen, hat es sich als vorteilhaft erwiesen, diese durch Zugabe der der nötigen Menge einer Base wie bevorzugt wässriger Kaliumhydroxid (KOH)-Lösung oder Natriumhydroxid-Lösung zu behandeln, wie es dem Fachmann bekannt ist.

Das erfindungsgemäße Hydrierverfahren ist ein selektives Verfahren zur Hydrierung organischer Verbindungen, die hydrierbare Gruppen enthalten, bevorzugt zur Hydrierung einer carbocyclischen aromatischen Gruppe zu der entsprechenden carbocyclischen aliphatischen Gruppe, mit dem hohe Ausbeuten und Raum-Zeit-Ausbeuten, [Produktmenge / (Katalysatorvolumen • Zeit)] (kg/(I_{Kat.} • h)), [Produktmenge / (Reaktorvolumen • Zeit)] (kg/(I_{Reaktor} • h)), bezogen auf den eingesetzten Katalysator erreicht werden können und in welchem die eingesetzten Katalysatoren ohne Aufarbeitung mehrfach für Hydrierungen eingesetzt werden können. Insbesondere werden in dem erfindungsgemäßen Hydrierverfahren lange Katalysatorstandzeiten erreicht.

Darüber hinaus eröffnet das erfindungsgemäße Hydrierverfahren einen leistungsfähigen und für Umsetzungen im technischen Maßstab geeigneten Zugang zu Isomerengemischen der genannten diastereomeren cycloaliphatischen Verbindungen der Formel (Ib) 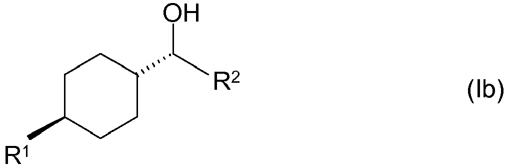 und der Formel (Ic)

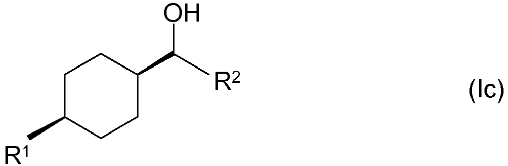

Die genannten Stereoisomeren werden im Rahmen des erfindungsgemäßen Verfahrens in der Regel in Form von Gemischen der jeweiligen cis- und trans-Isomere erhalten, wobei das relative Verhältnis von cis/trans oft im Bereich von 1,9:1 bis 2,5:1, bevorzugt im Bereich von 2,0:1 bis 2,4:1, noch mehr bevorzugt im Bereich von 2,1:1 bis 2,3:1 beträgt (Verhältnisse jeweils in mol/mol).

Dies ist insbesondere im Fall der erfindungsgemäßen Umsetzung des Cuminaldehyds der Formel (IIa) von Bedeutung, bei der das trans-Isomere der Formel (Id) 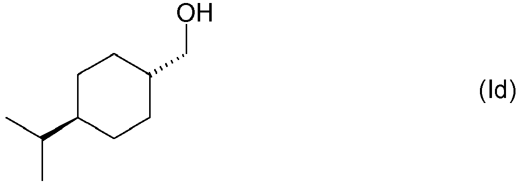 und das cis-Isomere der Formel (Ie) 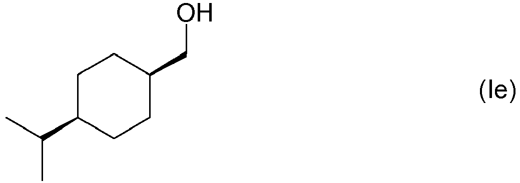 im vorstehend beschriebenen relativen Mengenverhältnis erhalten werden. Dies ist insbesondere im Hinblick auf eine Nutzung des so erhaltenen Gemisches als Aromachemikalie von Bedeutung, da sich die genannten Stereoisomere hinsichtlich ihres geruchlichen Eindruckes deutlich unterscheiden.

Das erfindungsgemäße Verfahren liefert daher einen industriell in besonders vorteilhafter Weise anwendbaren Zugang zu den als Aromachemikalien sehr gefragten Isomerengemischen der Formeln (Id) und (Ie). Dabei lässt sich die Zusammensetzung der erfindungsgemäß zugänglichen Produkte bezüglich ihres cis/trans-Verhältnisses durch geeignete Wahl der Reaktionsbedingungen in den einzelnen Hydrierreaktionen im Rahmen der vorstehenden Bereiche steuern, wodurch eine anschließende An- bzw. Abreicherung beispielsweise durch aufwendige destillative Trennverfahren häufig eingespart werden kann.

Die folgenden Beispiele dienen der Erläuterung der Erfindung, ohne sie jedoch in irgendeiner Weise zu beschränken:

### Beispiel 1:

### Herstellung eines auf SiO2-geträgerten Rutheniumkatalysators

50 kg eines SiO₂-Trägers (D11-10 (BASF SE); 3 mm Stränge, Wasseraufnahme von 0,95 ml/g, BET 135 m²/g) wurden in einer Tränktrommel vorgelegt und bei 96 bis 98 Gew.-% Wasseraufnahme getränkt. Die wässrige Tränklösung enthielt 0,176 kg Ru als Ru-Acetat (Umicore, 4,34 Gew.-% Ru). Der getränkte Katalysator wurde unbewegt bei einer Ofentemperatur von 145°C getrocknet bis zu einer Restfeuchte von circa 1 %. Die Reduktion erfolgte in Wasserstoff bewegt (circa 75 % H₂ in N₂, wobei N₂ als Spülstrom angewendet wird; 1,5 Nm³/h H₂ - 0,5 Nm³/h N₂) bei bewegter Schüttung bei 300°C und einer Verweilzeit von 90 Minuten (1-2 h). Die Passivierung erfolgte in verdünnter Luft (Luft in N₂). Die Luftzugabe wurde so geregelt, so dass die Temperatur des Katalysators unter 30 bis 35°C blieb. Der fertige Katalysator enthielt 0,31 bis 0,32 Gew.-% Ru.

### Beispiel 2:

Eine kontinuierlich betriebene Anlage bestehend aus zwei hintereinander geschalteten Rohrreaktoren (Hauptreaktor 150 mL und Nachreaktor 100 mL) wurde mit dem gemäß Beispiel 1 hergestellten Katalysator (Hauptreaktor: 58 g, Nachreaktor: 38 g) befüllt. Der Hauptreaktor wurde in Rieselfahrweise mit Umlauf betrieben (Umlauf/Zulauf = 100/1), der Nachreaktor im geraden Durchgang in Sumpffahrweise. Der Cuminaldehyd (47 g/h) wurde mit reinem Wasserstoff bei einer mittleren Temperatur von 125°C im Hauptreaktor und 110°C im Nachreaktor und einem Druck von 200 bar durch die Reaktorkaskade gepumpt. Die Katalysatorbelastung betrug 0,31 kg C_{uminaldehyd}/L_{Kat.}×h. Die gaschromatographische Analyse (GC-Säule: RTX 35, Länge 30 m, Durchmesser 0,25 µm; Temperaturprogramm: von 80°C mit 2°C/min bis 120°C, von 120°C mit 5°C/min auf 250°C) des Reaktionsaustrages zeigte, dass der Cuminaldehyd zu 100% umgesetzt worden war, auch das Zwischenprodukt Cuminalkohol war gaschromatographisch nicht mehr im Austragsgemisch nachweisbar. Die Selektivität an cis/trans-4-Isopropylcylclohexylmethanol betrug 90,7 Fl.-%. Als Nebenkomponenten konnten ca. 9 Fl.-% Leichtsieder (Komponenten mit einem niedrigeren Siedepunkt als 4-Isopropylcyclo-hexylmethanol wie z.B p-Cymol, Cumol, 1-Isopropyl-4-methylcyclohexan (cis/trans) und Isopropylcyclohexan) nachgewiesen werden. Das cis/trans-Verhältnis des erhaltenen 4-Isopropylcyclohexylmethanols betrug 2,20:1.

### Beispiel 3 (Vergleichsbeispiel):

In einem 300 mL Druckreaktor wurden 4,5 g des gemäß Beispiel 1 hergestellten Katalysators in einem Katalysator-Einsatzkorb vorgelegt und mit 150 g Cuminaldehyd versetzt. Die Hydrierung wurde mit reinem Wasserstoff bei einem konstanten Druck von 200 bar und einer Temperatur von 180°C durchgeführt. Es wurde so lange hydriert, bis kein Wasserstoff mehr aufgenommen wurde (35 Stunden). Der Reaktor wurde anschließend entspannt. Der Umsatz an Cuminaldehyd betrug 100%, auch das Zwischenprodukt Cuminalkohol war gaschromatographisch nicht mehr im Austragsgemisch nachweisbar (GC-Säule: RTX 35, Länge 30 m, Schichtdicke 0,25 µm; Temperaturprogramm: von 80°C mit 2°C/min bis 120°C, von 120°C mit 5°C/min auf 250°C). Die Selektivität an cis/trans-4-Isopropylcylclohexylmethanol betrug 87,5 Fl.-%. Als Nebenkomponenten konnten ca. 11,5 Fl.-% Leichtsieder (Komponenten mit einem niedrigeren Siedepunkt als 4-Isopropylcyclohexylmethanol wie z.B p-Cymol, Cumol, 1-Isopropyl-4-methyl-cyclohexan (cis/trans) und Isopropylcyclohexan) nachgewiesen werden. Das cis/trans-Verhältnis des erhaltenen 4-Isopropylcyclohexylmethanols betrug 1,86:1.

## Patentansprüche

1. Kontinuierliches Verfahren zur Herstellung eines 1-Hydroxyalkyl-cyclohexans der Formel (I) 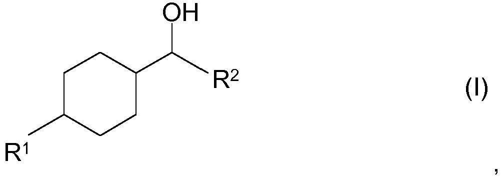 wobei die Reste
R¹ Isopropyl und
R² Wasserstoff oder einen geradkettigen oder verzweigten Alkylrest mit 1 bis 3 Kohlenstoffatomen
bedeuten,
durch katalytische Hydrierung einer aromatischen Carbonylverbindung der Formel (II) 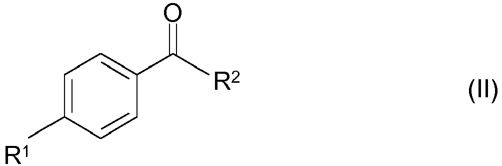 und/oder durch katalytische Hydrierung eines aromatischen Alkohols der Formel (III) 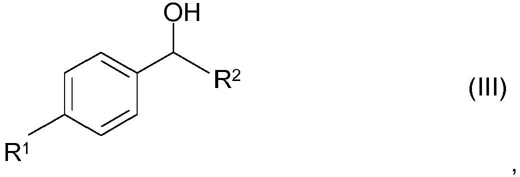 in denen die Reste R¹ und R² jeweils die gleiche Bedeutung wie in Formel (I) aufweisen,
in Gegenwart von Wasserstoff und in Gegenwart eines Katalysators, der als Aktivmetall Ruthenium aufgebracht auf einen Träger umfasst, wobei der Träger SiO₂ umfasst und wobei man den Katalysator in Form eines Festbettkatalysators in mehreren hintereinander geschalteten Hydrierreaktoren einsetzt, wobei der Festbettkatalysator bezogen auf das Gesamtgewicht des fertigen Katalysators einen Rutheniumgehalt von 0,1 bis 0,5 Gew.-% aufweist, wobei man die katalytische Hydrierung in einer Kaskade von n hintereinander geschalteten Hydrierreaktoren durchführt, wobei n eine ganze Zahl von 2 bis 5 bedeutet und wobei man
a1) das aus einem vorangehenden Hydrierreaktor kontinuierlich ausgetragene und nicht zurückgeführte Reaktionsgemisch und Wasserstoff kontinuierlich in den darauffolgenden Hydrierreaktor einträgt,
b1) das erhaltene, 1-Hydroxyalkyl-cyclohexan der Formel (I) enthaltende Reaktionsgemisch aus dem jeweiligen Hydrierreaktor kontinuierlich austrägt und gewünschtenfalls teilweise wieder in den jeweiligen Hydrierreaktor zurückführt und
c1) gewünschtenfalls das 1-Hydroxyalkyl-Cyclohexan der Formel (I) von dem in Schritt b1) aus dem n-ten Hydrierreaktor ausgetragenen und nicht wieder zurückgeführten Reaktionsgemisch abtrennt.

2. Verfahren nach Anspruch 1, **dadurch gekennzeichnet, dass** man
a) die Ausgangsverbindungen der Formel (II) und/oder (III) sowie Wasserstoff kontinuierlich in den ersten Hydrierreaktor einträgt,
b) das erhaltene, 1-Hydroxyalkyl-cyclohexan der Formel (I) umfassende Reaktionsgemisch kontinuierlich aus dem ersten Hydrierreaktor austrägt und gewünschtenfalls teilweise wieder in den ersten Hydrierreaktor zurückführt und
c) gewünschtenfalls das 1-Hydroxyalkyl-Cyclohexan der Formel (I) von dem in Schritt b) aus dem ersten Hydrierreaktor ausgetragenen und nicht wieder zurückgeführten Reaktionsgemisch abtrennt.

3. Verfahren nach einem der Ansprüche 1 oder 2, **dadurch gekennzeichnet, dass** man eine aromatische Carbonylverbindung der Formel (II) einsetzt.

4. Verfahren nach einem der Ansprüche 1 bis 3, **dadurch gekennzeichnet, dass** man einen Festbettkatalysator einsetzt, der lediglich SiO₂ als Träger umfasst.

5. Verfahren nach einem der Ansprüche 1 bis 4, **dadurch gekennzeichnet, dass** man einen Festbettkatalysator einsetzt, enthaltend als Aktivmetall Ruthenium alleine oder zusammen mit mindestens einem weiteren Metall der Nebengruppen IB, VIIB oder VIII des Periodensystems der Elemente, aufgebracht auf einen Träger enthaltend Siliziumdioxid als Trägermaterial, wobei die Menge des Aktivmetalls < 1 Gew.-% beträgt, bezogen auf das Gesamtgewicht des Katalysators, und mindestens 60 Gew.-% des Aktivmetalls in der Schale des Katalysators bis zu einer Eindringtiefe von 200 µm vorliegen, ermittelt mittels SEM-EPMA.

6. Verfahren nach einem der Ansprüche 1 bis 5, **dadurch gekennzeichnet, dass** man die Hydrierung ohne Zusatz von Lösungsmitteln durchführt.

7. Verfahren nach einem der Ansprüche 1 bis 6, **dadurch gekennzeichnet, dass** man den ersten Hydrierreaktor in Rieselfahrweise betreibt.

8. Verfahren nach einem der Ansprüche 1 bis 7, **dadurch gekennzeichnet, dass** man den weiteren Hydrierreaktor oder mindestens einen der weiteren Hydrierreaktoren in Sumpffahrweise betreibt.

9. Verfahren nach einem der Ansprüche 1 bis 8, **dadurch gekennzeichnet, dass** man die Hydrierung bei Drücken im Bereich von 100 bis 250 bar durchführt.

10. Verfahren nach einem der Ansprüche 1 bis 9, **dadurch gekennzeichnet, dass** man die Hydrierung bei Temperaturen im Bereich von 80 bis 160°C durchführt.

11. Verfahren nach einem der Ansprüche 1 bis 10, **dadurch gekennzeichnet, dass** man einen aromatischen Aldehyd der Formel (II) einsetzt, der einen Säurewert von 0 bis 5 mg KOH/g aufweist.

## Claims

1. A continuous process for preparing a 1-hydroxyalkylcyclohexane of the formula (I) 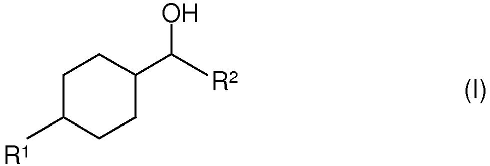 where
R¹ is isopropyl and
R² is hydrogen or a straight-chain or branched alkyl radical having from 1 to 3 carbon atoms,
by catalytically hydrogenating an aromatic carbonyl compound of the formula (II) 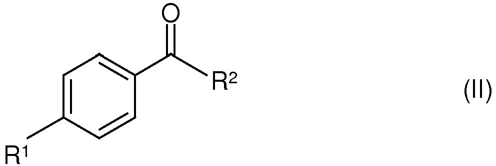 and/or by catalytically hydrogenating an aromatic alcohol of the formula (III) 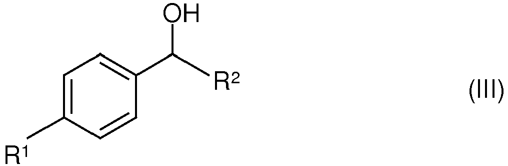 in which the R¹ and R² radicals are each as defined in formula (I),
in the presence of hydrogen and in the presence of a catalyst which comprises, as an active metal, ruthenium applied to a support, the support comprising SiO₂, and the catalyst being used in the form of a fixed bed catalyst in a plurality of hydrogenation reactors connected in series, the fixed bed catalyst, based on the total weight of the finished catalyst, having a ruthenium content of from 0.1 to 0.5% by weight, the catalytic hydrogenation being performed in a cascade of n hydrogenation reactors connected in series, where n is an integer from 2 to 5, and where
a1) the reaction mixture which has been discharged continuously from an upstream hydrogenation reactor and not recycled, and hydrogen, are introduced continuously into the downstream hydrogenation reactor,
b1) the resulting reaction mixture comprising 1-hydroxyalkylcyclohexane of the formula (I) is discharged continuously from the particular hydrogenation reactor and, if desired, partly recycled back into the particular hydrogenation reactor, and
c1) if desired, the 1-hydroxyalkylcyclohexane of the formula (I) is removed from the reaction mixture which has been discharged from the nth hydrogenation reactor in step b1) and not recycled.

2. The process according to claim 1, wherein
a) the starting compounds of the formula (II) and/or (III) and hydrogen are introduced continuously into the first hydrogenation reactor,
b) the resulting reaction mixture comprising 1-hydroxyalkylcyclohexane of the formula (I) is discharged continuously from the first hydrogenation reactor and, if desired, partly recycled back into the first hydrogenation reactor and
c) if desired, the 1-hydroxyalkylcyclohexane of the formula (I) is removed from the reaction mixture which has been discharged from the first hydrogenation reactor in step b) and not recycled.

3. The process according to either of claims 1 and 2, wherein an aromatic carbonyl compound of the formula (II) is used.

4. The process according to any one of claims 1 to 3, wherein a fixed bed catalyst which comprises only SiO₂ as a support is used.

5. The process according to any one of claims 1 to 4, wherein a fixed bed catalyst comprising, as an active metal, ruthenium alone or together with at least one further metal of transition groups IB, VIIB or VIII of the Periodic Table of the Elements, applied to a support comprising silicon dioxide as a support material, the amount of the active metal being < 1% by weight, based on the total weight of the catalyst, and at least 60% by weight of the active metal being present in the coating of the catalyst up to a penetration depth of 200 µm, determined by means of SEM-EPMA, is used.

6. The process according to any one of claims 1 to 5, wherein the hydrogenation is performed without addition of solvents.

7. The process according to any one of claims 1 to 6, wherein the first hydrogenation reactor is operated in trickle mode.

8. The process according to any one of claims 1 to 7, wherein the further hydrogenation reactor or at least one of the further hydrogenation reactors is operated in liquid phase mode.

9. The process according to any one of claims 1 to 8, wherein the hydrogenation is performed at pressures in the range from 100 to 250 bar.

10. The process according to any one of claims 1 to 9, wherein the hydrogenation is performed at temperatures in the range from 80 to 160°C.

11. The process according to any one of claims 1 to 10, wherein an aromatic aldehyde of the formula (II) which has an acid value of from 0 to 5 mg KOH/g is used.

## Revendications

1. Procédé continu pour la préparation d'un 1-hydroxyalkylcyclohexane de formule (I) 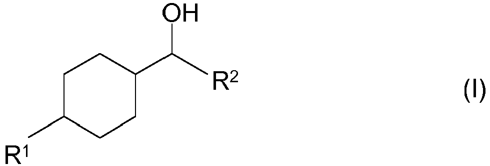 dans laquelle
R¹ signifie isopropyle et
R² signifie hydrogène ou un radical alkyle linéaire ou ramifié comprenant 1 à 3 atomes de carbone,
par hydrogénation catalytique d'un composé carbonyle aromatique de formule (II) 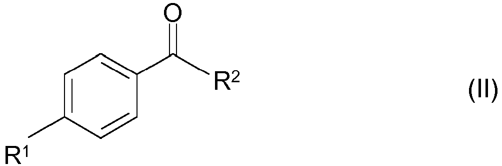 et/ou par hydrogénation catalytique d'un alcool aromatique de formule (III) 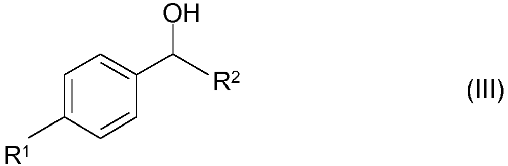 dans laquelle les radicaux R¹ et R² présentent à chaque fois la même signification que dans la formule (I),
en présence d'hydrogène et en présence d'un catalyseur qui comprend, comme métal actif, du ruthénium appliqué sur un support, le support comprenant du SiO₂ et le catalyseur étant utilisé sous forme d'un catalyseur à lit fixe dans plusieurs réacteurs d'hydrogénation disposés les uns derrière les autres, le catalyseur à lit fixe présentant, par rapport au poids total du catalyseur fini, une teneur en ruthénium de 0,1 à 0,5% en poids, l'hydrogénation catalytique étant réalisée dans une cascade de n réacteurs disposés les uns derrière les autres, n signifiant un nombre entier de 2 à 5 et
a1) le mélange réactionnel et l'hydrogène évacués d'un réacteur d'hydrogénation préalable et non recyclés étant introduits dans le réacteur d'hydrogénation suivant,
b1) le mélange réactionnel obtenu, contenant du 1-hydroxyalkylcyclohexane de formule (I), étant évacué en continu du réacteur d'hydrogénation respectif et, le cas échéant, recyclé de nouveau en partie dans le réacteur d'hydrogénation respectif et
c1) le cas échéant, le 1-hydroxyalkylcyclohexane de formule (I) étant séparé du mélange réactionnel évacué dans l'étape b1) du nième réacteur d'hydrogénation et non recyclé.

2. Procédé selon la revendication 1, **caractérisé en ce qu'**on
a) introduit les composés de départ de (II) et/ou (III) ainsi que de l'hydrogène en continu dans le premier réacteur d'hydrogénation,
b) évacue le mélange réactionnel obtenu, comprenant du 1-hydroxyalkylcyclohexane de formule (I), en continu du premier réacteur d'hydrogénation et le recycle le cas échéant de nouveau en partie dans le premier réacteur d'hydrogénation et
c) le cas échéant, sépare le 1-hydroxyalkylcyclohexane de formule (I) du mélange réactionnel évacué dans l'étape b) du premier réacteur d'hydrogénation et non recyclé.

3. Procédé selon l'une quelconque des revendications 1 ou 2, **caractérisé en ce qu'**on utilise un composé carbonyle aromatique de formule (II).

4. Procédé selon l'une quelconque des revendications 1 à 3, **caractérisé en ce qu'**on utilise un catalyseur à lit fixe qui ne comprend que du SiO₂ comme support.

5. Procédé selon l'une quelconque des revendications 1 à 4, **caractérisé en ce qu'**on utilise un catalyseur à lit fixe, contenant, comme métal actif, du ruthénium seul ou ensemble avec au moins un autre métal des groupes secondaires IB, VIIB ou VIII du système périodique des éléments, appliqué sur un support contenant du dioxyde de silicium comme matériau support, la quantité de métal actif étant < 1% en poids, par rapport au poids total du catalyseur, et au moins 60% en poids du métal actif se trouvant dans la coquille du catalyseur jusqu'à une profondeur de pénétration de 200 µm, déterminée par SEM-EPMA.

6. Procédé selon l'une quelconque des revendications 1 à 5, **caractérisé en ce qu'**on réalise l'hydrogénation sans addition de solvants.

7. Procédé selon l'une quelconque des revendications 1 à 6, **caractérisé en ce que** le premier réacteur d'hydrogénation est exploité dans un mode par ruissellement.

8. Procédé selon l'une quelconque des revendications 1 à 7, **caractérisé en ce qu'**on exploite l'autre réacteur d'hydrogénation ou moins un des autres réacteurs d'hydrogénation dans un mode avec un résidu.

9. Procédé selon l'une quelconque des revendications 1 à 8, **caractérisé en ce qu'**on réalise l'hydrogénation à des pressions dans la plage de 100 à 250 bars.

10. Procédé selon l'une quelconque des revendications 1 à 9, **caractérisé en ce qu'**on réalise l'hydrogénation à des températures dans la plage de 80 à 160°C.

11. Procédé selon l'une quelconque des revendications 1 à 10, **caractérisé en ce qu'**on utilise un aldéhyde aromatique de formule (II), qui présente une valeur d'acide de 0 à 5 mg de KOH/g.
